# EUROPEAN PATENT SPECIFICATION

(11) **EP 3 818 964 B1**
(45) Date of publication and mention of the grant of the patent: **10.07.2024**
(21) Application number: 19207648.7
(22) Date of filing: 07.11.2019
(51) Int. Cl.: A61F 2/44, A61F 2/46

(54) **MULTI-SECTION EXPANDABLE DEVICE**
MEHRTEILIGE EXPANDIERBARE VORRICHTUNG
DISPOSITIF EXTENSIBLE À PLUSIEURS SECTIONS

(43) Date of publication of application: 12.05.2021
(73) Proprietor: OSSAWARE Biotech Co., Ltd., Changhua County 509 (TW)
(72) Inventor: HUANG, Max, 509 Shengang Township, Changhua County (TW)
(74) Representative: Lucke, Andreas

(56) References cited:
- US-A1- 2014 094 916
- US-A1- 2018 193 164
- US-A1- 2019 269 521

## Description

### FIELD OF THE INVENTION

The present invention relates to an expandable implant, and more particularly to a multi-section expandable device. Regardless of how large the distance between the upper and lower vertebrae is, the multi-section expandable device can expand to have a larger width, thereby providing a better support effect and avoiding the wear of the contact faces of the vertebrae.

### BACKGROUND OF THE INVENTION

At present, the minimally invasive surgery of intervertebral fusion is mainly to create a tiny channel in the affected part, and then an intervertebral infusion device is implanted to be between the upper and lower vertebrae of the affected part using the endoscope or X-ray. The intervertebral infusion device is longitudinally expanded to abut against the opposite end faces of the upper and lower vertebrae for accomplishing the intervertebral fusion.

A conventional intervertebral infusion device, as disclosed in U.S. Patent No. 9,883,955 B2 and U.S. Patent No. 10,045,858 B2, can be expanded in one direction only. When the intervertebral infusion device is longitudinally implanted between the upper and lower vertebrae, it can expand longitudinally to support the upper and lower vertebrae. Since the contact area between the intervertebral infusion device and the upper and lower vertebrae is very small, the end faces of the upper and lower vertebrae are worn easily to cause a depression. When the intervertebral infusion device is laterally implanted between the upper and lower vertebrae, it can expand laterally to increase the width so as to increase the contact area, thereby improving the wear and depression of the vertebrae. However, the height is immovable and cannot be adjusted. Therefore, once the distance between the upper and lower vertebrae of the patient is large, the intervertebral infusion device is unable to abut against the opposite end faces of the upper and lower vertebrae, failing to accomplish the intervertebral fusion.

Another conventional intervertebral infusion device, as disclosed in U.S. Patent No. 8,864,833 B2 and U.S. Patent No. 10,098,758 B2, can be expanded longitudinally and transversely. However, because the size of the human body, the severity of the disease and the affected part are different, the required height of the longitudinal expansion of the intervertebral infusion device will be different. Therefore, when the intervertebral infusion device is implanted between the upper and lower vertebrae that need a large longitudinal expansion, it can be expanded longitudinally and laterally to the maximum, not having the problem that the end faces of the upper and lower vertebrae are worn easily to cause a depression. However, when the intervertebral infusion device is implanted between the upper and lower vertebrae that need a small longitudinal expansion, the distance of the longitudinal expansion is limited and only small or even no. The lateral expansion is linked with the longitudinal expansion, so the distance of the lateral expansion will be relatively small or even no. There is still a problem that the end faces of the upper and lower vertebrae are worn easily to cause a depression because the width is not enough. Furthermore, the longitudinal expansion and the lateral expansion are performed at the same time to cause a relatively large operational resistance, and thus there are problems such as poor smoothness and laboriousness.

Further embodiments of an expandable device are disclosed in US 2018/193 164 A1, US 2014/094 916 A1, and US 2019/269 521 A1.

### SUMMARY OF THE INVENTION

According to the present invention, a multi-section expandable device according to claim 1 is provided. Further developments of a multi-section expandable device are the subject matter of the dependent claims.

The primary object of the present invention is to provide a multi-section expandable device, comprising an expansion module, a first push member, a second push member, and a bolt. The expansion module is able to expand longitudinally and laterally. The first push member is disposed at a front end of the expansion module and has a screw hole therein. The second push member is disposed at a rear end of the expansion module and has a through hole therein. The bolt has a screw portion and a head portion with a larger outer diameter. The screw portion is inserted through the through hole of the second push member and screwed in the screw hole of the first push member. The head portion abuts against one side of the second push member opposite to the first push member. When the bolt is tightened, the first push member and the second push member are pushed to approach each other so as to push the expansion module to generate a first-stage expansion and a second-stage expansion. The first-stage expansion enables the expansion module to expand laterally so as to adjust its width. The second-stage expansion is performed after the expansion module is laterally expanded to have a maximum width, so that the expansion module is longitudinally expanded to adjust its height.

The multi-section expandable device provided by the present invention firstly performs the first-stage expansion for the expansion module to expand laterally to have the maximum width, and then performs the second-stage expansion for the expansion module to expand longitudinally to adjust its height. Therefore, no matter how much height is required between the upper and lower vertebrae, the expansion module can be expanded laterally to have the maximum width and a large contact area. It not only has better support effect, but also avoids the wear of the contact surfaces of the vertebrae. The multi-section expandable device with a single specification can be applied to the intervertebral disc space of various body sizes, different diseases and different affected parts. Moreover, the two-stage expansion can greatly reduce the operational resistance, thereby increasing the smoothness and being labor-saving.

### BRIEF DESCRIPTION OF THE DRAWINGS

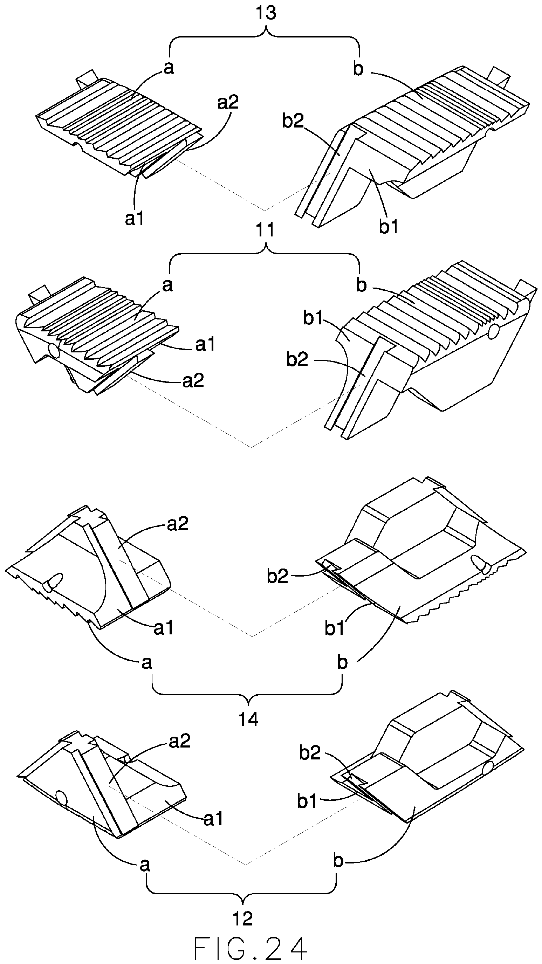

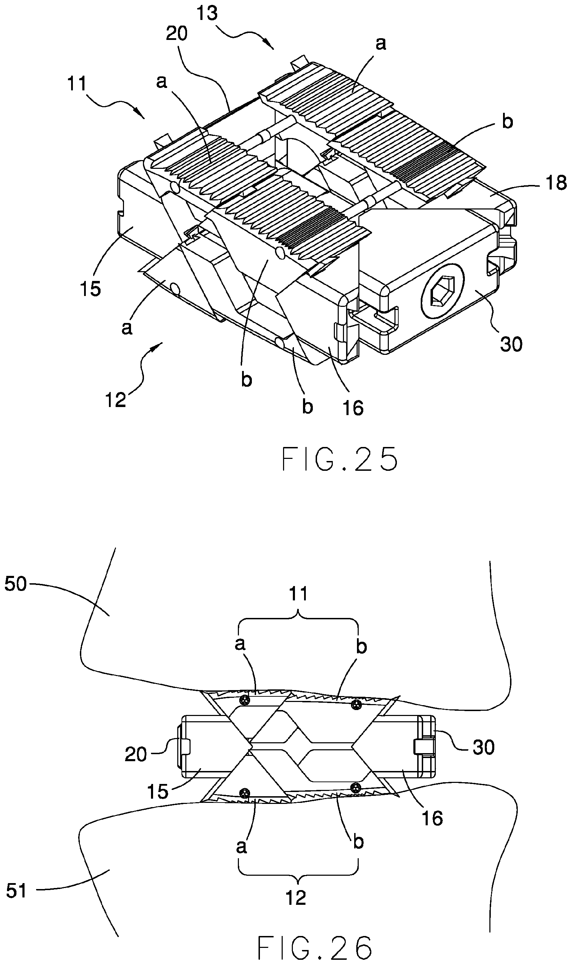

The embodiments of the invention are set forth as "fourth embodiment of the invention" and illustrated in fig. 24 to 26. The examples of the present disclosure set forth as "first", "second" and "third embodiment" shown in the other figures do not form part of the invention but represent background art that is useful for understanding the invention.
FIG. 1 is an exploded view in accordance with a first embodiment;
FIG. 2 is another exploded view in accordance with the first embodiment;
FIG. 3 is a perspective view in accordance with the first embodiment;
FIG. 4 is a top view of FIG. 3;
FIG. 5 is a front view of FIG. 3;
FIG. 6 is a right side view of FIG. 3;
FIG. 7 is a cross-sectional view taken along line A-A of FIG. 5;
FIG. 8 is a cross-sectional view taken along line B-B of FIG. 5;
FIG. 9 is a perspective view showing the first-stage expansion in accordance with the first embodiment;
FIG. 10 is front schematic view showing the first-stage expansion in accordance with the first embodiment;
FIG. 11 is an enlarged cross-sectional view taken along line C-C of FIG. 10;
FIG. 12 is a perspective view showing the second-stage expansion in accordance with the first embodiment;
FIG. 13 is front schematic view showing the second-stage expansion in accordance with the first embodiment;
FIG. 14 is an enlarged cross-sectional view taken along line D-D of FIG. 13;
FIG. 15 is an exploded view in accordance with a second embodiment;
FIG. 16 is another exploded view in accordance with the second embodiment;
FIG. 17 is a perspective view in accordance with the second embodiment in a fully retracted state;
FIG. 18 is a perspective view showing the first-stage expansion to the ultimate in accordance with the second embodiment;
FIG. 19 is a perspective view in accordance with the second embodiment in a fully expanded state;
FIG. 20 is a cross-sectional view showing the first-stage expansion to the ultimate in accordance with the second embodiment;
FIG. 21 is a schematic view showing the operation of the limit structure in accordance with the second embodiment;
FIG. 22 is a schematic view showing the operation of the guide structure in accordance with the second embodiment;
FIG. 23 is an exploded view in accordance with a embodiment;
FIG. 24 is a partial enlarged view in accordance with a fourth embodiment of the present invention;
FIG. 25 is a perspective view showing the second-stage longitudinal expansion in accordance with the fourth embodiment of the present invention;
FIG. 26 is a schematic view showing the second-stage longitudinal expansion in accordance with the fourth embodiment of the present invention when in use; and
FIG. 27 is a schematic view of the present invention implanted in a bone.

### DETAILED DESCRIPTION OF THE PREFERRED EMBODIMENTS

Embodiments of the present invention will now be described, by way of example only, with reference to the accompanying drawings.

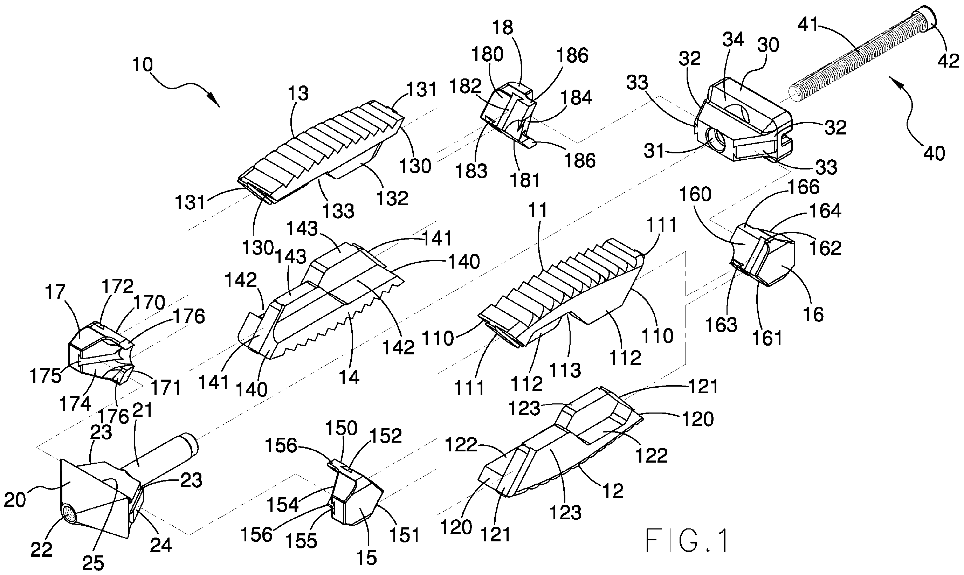

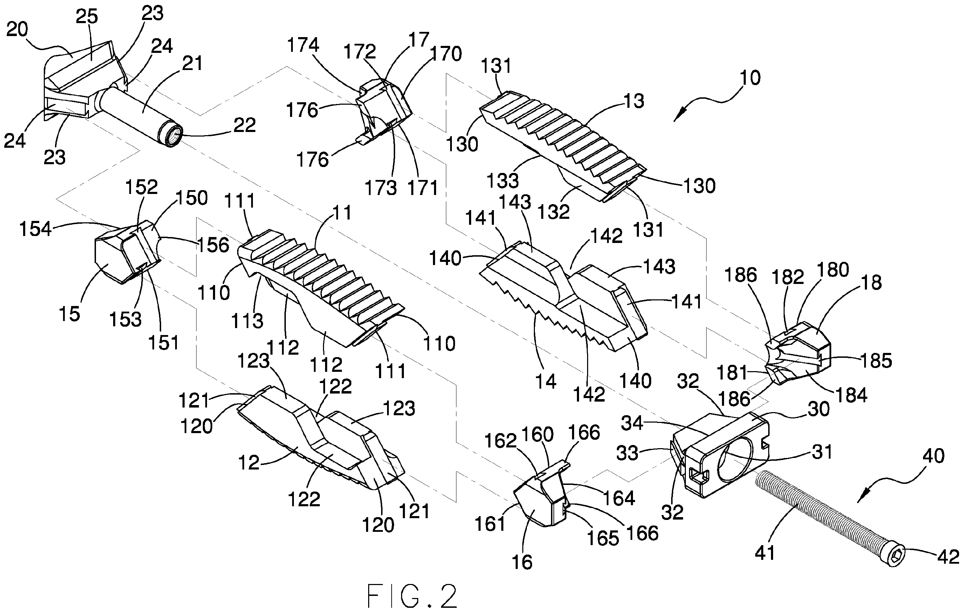

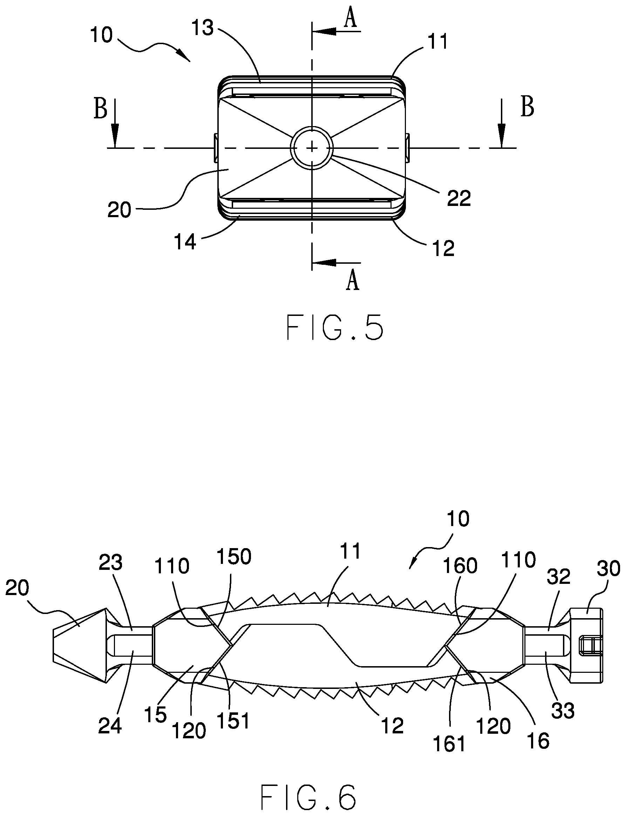

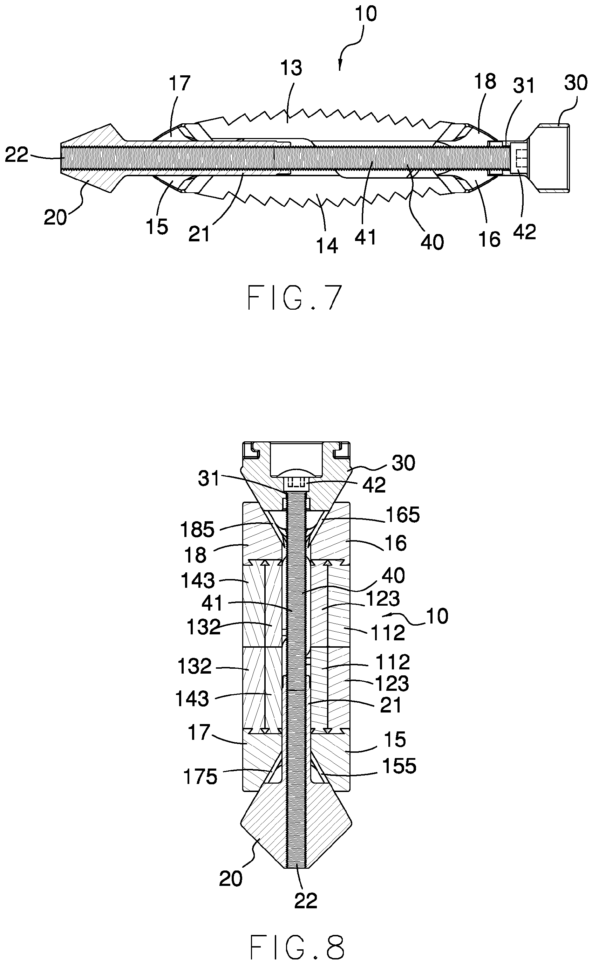

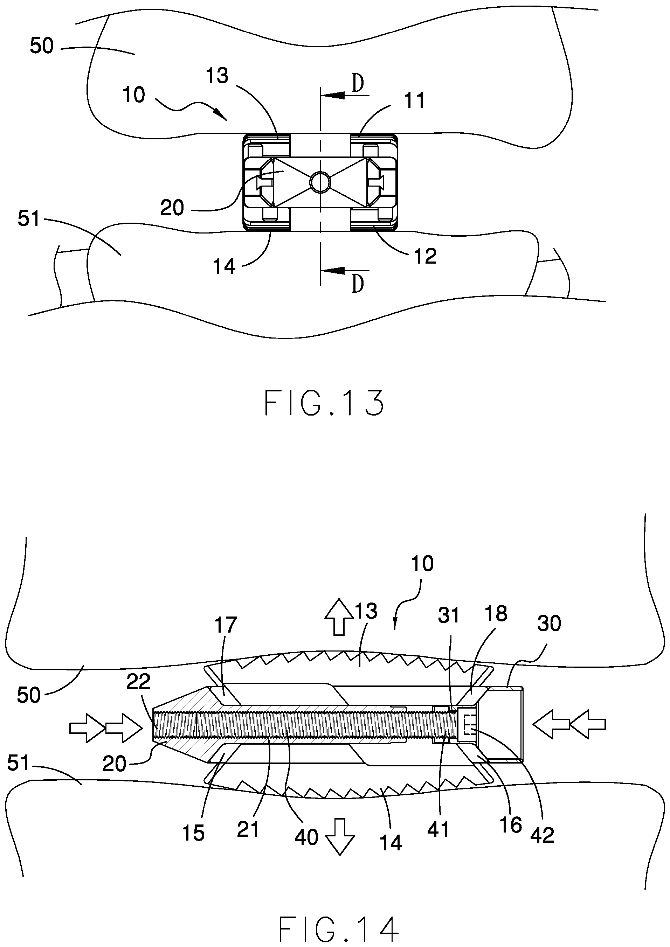

FIGS. 1-14 illustrate a two-stage expandable intervertebral fusion device in accordance with a first embodiment.

As shown in FIGS. 1-8, the multi-section expandable device comprises an expansion module 10, a first push member 20, a second push member 30, and a bolt 40.

The expansion module 10 is able to expand longitudinally and laterally.

The first push member 20 is disposed at a front end of the expansion module 10. A rod 21 is protruded from a rear side of the first push member 20. The rod 21 has a screw hole 22 therein.

The second push member 30 is disposed at a rear end of the expansion module 10. The second push member 30 has a through hole 31 therein.

The bolt 40 has a screw portion 41 and a head portion 42 with a larger outer diameter. The screw portion 41 is inserted through the through hole 31 of the second push member 30 and screwed in the screw hole 22 of the first push member 20. The head portion 42 abuts against one side of the second push member 30 opposite to the first push member 20.

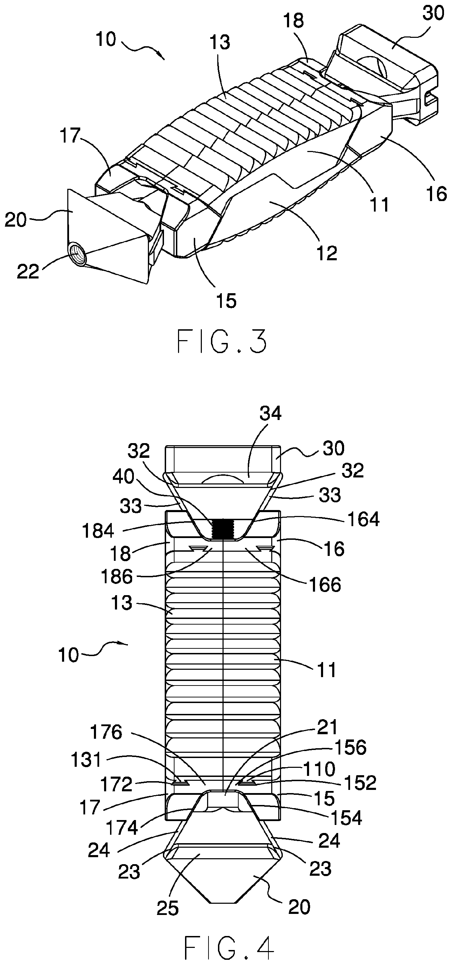

Referring to the FIG. 3, FIG. 9 and FIG. 12, when the bolt 40 is tightened, the first push member 20 and the second push member 30 are pushed to approach each other so as to push the expansion module 10 to generate a first-stage expansion and a second-stage expansion, the first-stage expansion enables the expansion module 10 to expand laterally to adjust its width, the second-stage expansion is performed after the expansion module 10 is laterally expanded to have a maximum width, so that the expansion module 10 can be longitudinally expanded to adjust its height as needed.

The expansion module 10 includes a first expansion block 11, a second expansion block 12, a third expansion block 13, a fourth expansion block 14, a first guide block 15, a second guide block 16, a third guide block 17, and a fourth guide block 18.

The first expansion block 11 has a toothed top surface. Front and rear ends of the first expansion block 11 are formed with two first inclined planes 110 that are inclined outwardly from bottom to top, respectively. Two first inclined plane guide rails 111 are disposed on the two first inclined planes 110, respectively. A bottom surface of the first expansion block 11 has a plurality of first guide protrusions 112 and a plurality of first guide grooves 113 which are staggered and each have a trapezoidal shape.

The second expansion block 12 is located under the first expansion block 11 and has a toothed button surface. Front and rear ends of the second expansion block 12 are formed with two second inclined planes 120 that are inclined outwardly from top to bottom, respectively. Two second inclined plane guide rails 121 are disposed on the two second inclined planes 120, respectively. A top surface of the second expansion block 12 has a plurality of second guide grooves 122 and a plurality of second guide protrusions 123 which can be slid up and down relative to the plurality of first guide protrusions 112 and the plurality of first guide grooves 113.

The third expansion block 13 is located beside the first expansion block 11 has a toothed top surface. Front and rear ends of the third expansion block 13 are formed with two third inclined planes 130 that are inclined outwardly from bottom to top, respectively. Two second inclined plane guide rails 113 are disposed on the two second inclined planes 130, respectively. A bottom surface of the third expansion block 13 has a plurality of third guide protrusions 132 and a plurality of third guide grooves 133 which are staggered and each have a trapezoidal shape.

The fourth expansion block 14 is located under the third expansion block 13 and has a toothed button surface. Front and rear ends of the fourth expansion block 14 are formed with two fourth inclined planes 140 that are inclined outwardly from top to bottom, respectively. Two fourth inclined plane guide rails 141 are disposed on the two fourth inclined planes 140, respectively. A top surface of the fourth expansion block 14 has a plurality of fourth guide grooves 142 and a plurality of fourth guide protrusions 143 which can be slid up and down relative to the plurality of third guide protrusions 132 and the plurality of third guide grooves 133.

The first guide block 15 is disposed on the front end faces of the first and second expansion blocks 11, 12. A rear end of the first guide block 15 is formed with a first upper inclined plane 150 and a first lower inclined plane 151 corresponding to the first and second inclined planes 110, 120 on the front ends of the first and second expansion blocks 11, 12. The first upper inclined plane 150 is provided with a first upper dovetailed groove 152 for the first inclined plane guide rail 111 on the front end of the first expansion block 11 to be inserted therein. The first lower inclined plane 151 is provided with a first lower dovetailed groove 153 for the second inclined plane guide rail 121 on the front end of the second expansion block 12 to be inserted therein. A front end of the first guide block 15 is formed with a first front inclined plane 154 that is inclined outwardly from back to front. The first front inclined plane 154 is provided with a first front dovetailed groove 155. Two first front stop pieces 156 are protruded from a lower end of the first front inclined plane 154.

The second guide block 16 is disposed on the rear end faces of the first and second expansion blocks 11, 12. A front end of the second guide block 16 is formed with a second upper inclined plane 160 and a second lower inclined plane 161 corresponding to the first and second inclined planes 110, 120 on the rear ends of the first and second expansion blocks 11, 12. The second upper inclined plane 160 is provided with a second upper dovetailed groove 162 for the first inclined plane guide rail 111 on the rear end of the first expansion block 11 to be inserted therein. The second lower inclined plane 161 is provided with a second lower dovetailed groove 163 for the second inclined plane guide rail 121 on the rear end of the second expansion block 12 to be inserted therein. A rear end of the second guide block 16 is formed with a second rear inclined plane 164 that is inclined outwardly from front to back. The second rear inclined plane 164 is provided with a second rear dovetailed groove 165. Two second rear stop pieces 166 are protruded from a lower end of the second rear inclined plane 164.

The third guide block 17 is disposed on the front end faces of the third and fourth expansion blocks 13, 14. A rear end of the third guide block 17 is formed with a third upper inclined plane 170 and a third lower inclined plane 171 corresponding to the third and fourth inclined planes 130, 140 on the front ends of the third and fourth expansion blocks 13, 14. The third upper inclined plane 170 is provided with a third upper dovetailed groove 172 for the third inclined plane guide rail 131 on the front end of the third expansion block 13 to be inserted therein. The third lower inclined plane 171 is provided with a third lower dovetailed groove 173 for the fourth inclined plane guide rail 141 on the front end of the fourth expansion block 14 to be inserted therein. A front end of the third guide block 17 is formed with a third front inclined plane 174 that is inclined outwardly from back to front. The third front inclined plane 174 is provided with a third front dovetailed groove 175. Two third front stop pieces 176 are protruded from a lower end of the third front inclined plane 174.

The fourth guide block 18 is disposed on the rear end faces of the third and fourth expansion blocks 13, 14. A front end of the fourth guide block 18 is formed with a fourth upper inclined plane 180 and a fourth lower inclined plane 181 corresponding to the third and fourth inclined planes 130, 140 on the rear ends of the third and fourth expansion blocks 13, 14. The fourth upper inclined plane 180 is provided with a fourth upper dovetailed groove 182 for the third inclined plane guide rail 131 on the rear end of the third expansion block 13 to be inserted therein. The fourth lower inclined plane 181 is provided with a fourth lower dovetailed groove 183 for the fourth inclined plane guide rail 141 on the rear end of the fourth expansion block 14 to be inserted therein. A rear end of the fourth guide block 18 is formed with a fourth rear inclined plane 184 that is inclined outwardly from front to back. The fourth front inclined plane 184 is provided with a fourth rear dovetailed groove 185. Two fourth rear stop pieces 186 are protruded from a lower end of the fourth rear inclined plane 184.

Two sides of the first push member 20 have two first push member inclined planes 23 corresponding to the first and third front inclined planes 154, 174. The two first push member inclined planes 23 are provided with two first push member guide rails 24 to be inserted in the first and third front dovetailed grooves 155, 175. The first push member 20 has two first stop faces 25 on upper and lower sides thereof.

Two sides of the second push member 30 have two second push member inclined planes 32 corresponding to the second and fourth rear inclined planes 164, 184. The two second push member inclined planes 32 are provided with two second push member guide rails 33 to be inserted in the second and fourth rear dovetailed grooves 165, 185. The second push member 30 has two second stop faces 34 on upper and lower sides thereof.

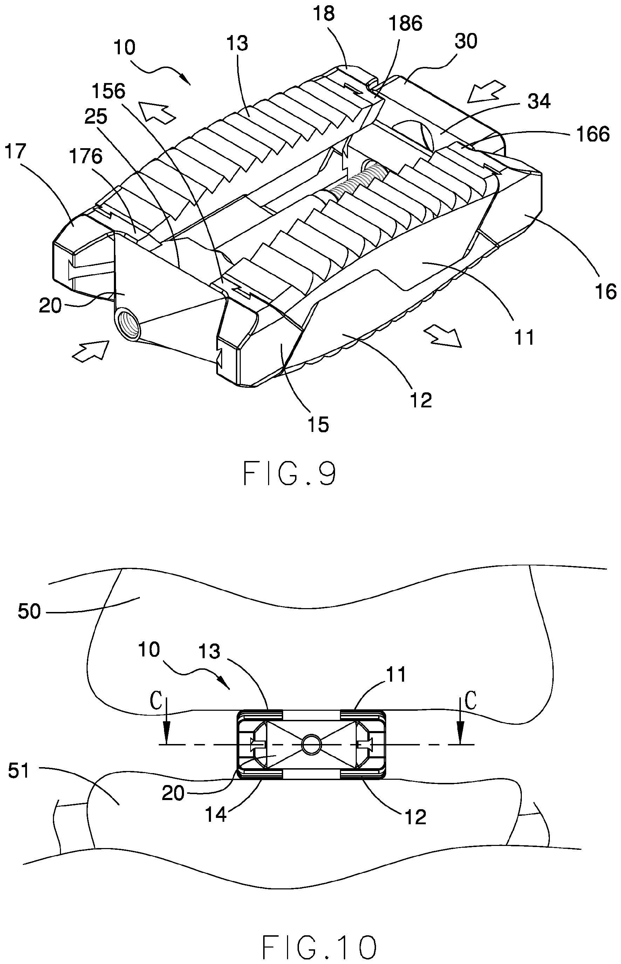

FIGS. 9-11 illustrate the operation for the first-stage expansion of the multi-section expandable device of the present disclosure. invention. When the bolt 40 is tightened, the first and second push members 20, 30 are pushed to approach each other to synchronously push the first guide block 15, the second guide block 16, the third guide block 17, and the fourth guide block 18. The first and second guide blocks 15, 16 and the third and fourth guide blocks 17, 18 are moved outwardly (left and right) along the two first push member inclined planes 23 of the first push member 20 and the two second push member inclined planes 32 of the second push member 30, respectively, so that the first and second guide blocks 15, 16 and the third and fourth guide blocks 17, 18 respectively drive the first and second expansion blocks 11, 12 and the third and fourth expansion blocks 13, 14 to expand laterally between the first and second push members 20, 30 so as to increase the width of the expansion module 10.

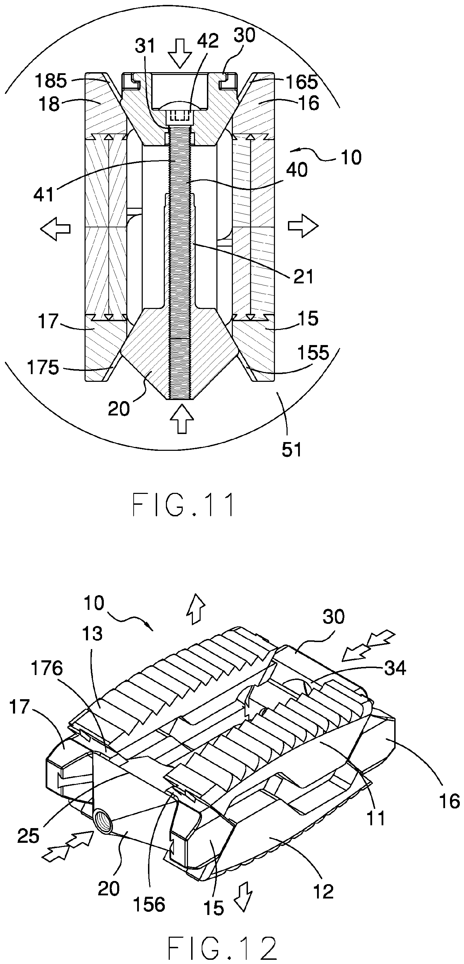

FIGS. 12-14 illustrate the operation for the second-stage expansion of the multi-section expandable device of the present dislcosure. When the two first stop faces 25 and the two second stop faces 34 are blocked by the two first front stop pieces 156, the two third front stop pieces 176 and the two second rear stop pieces 166, the two four rear stop pieces 186 respectively, the first and second expansion blocks 11, 12 and the third and fourth expansion blocks 13, 14 between the first and second push members 20, 30 can no longer continue to expand laterally. At this time, the expansion module 10 is laterally expanded to have the maximum width (this is, the ultimate width of the first-stage expansion). Then, the bolt 40 is further tightened, and the first and second expansion blocks 11, 12 are moved along the first upper inclined plane 150 and the first lower inclined plane 151 of the first guide block 15 and the second upper inclined plane 160 and the second lower inclined plane 161 of the second guide block 16 to expand longitudinally. At the same time, the third and fourth expansion blocks 13, 14 are also moved along the third upper inclined plane 170 and the third lower inclined plane 171 of the third guide block 17 and the fourth upper inclined plane 180 and the fourth lower inclined plane 181 of the fourth guide block 18 to expand longitudinally. Thus, the height of the expansion module 10 can be adjusted to lean against the upper vertebra 50 and the lower vertebra 51.

The multi-section expandable device provided by the present invention firstly performs the first-stage expansion for the expansion module to expand laterally to have the maximum width, and then performs the second-stage expansion for the expansion module to expand longitudinally to adjust its height. Therefore, no matter how much height is required between the upper and lower vertebrae, the expansion module can be expanded laterally to have the maximum width and a larger contact area. It not only has better support effect, but also avoids the wear of the contact surfaces of the vertebrae. The multi-section expandable device with a single specification can be applied to the intervertebral disc space of various body sizes, different diseases and different affected parts. Moreover, the two-stage expansion can greatly reduce the operational resistance, thereby increasing the smoothness and being labor-saving.

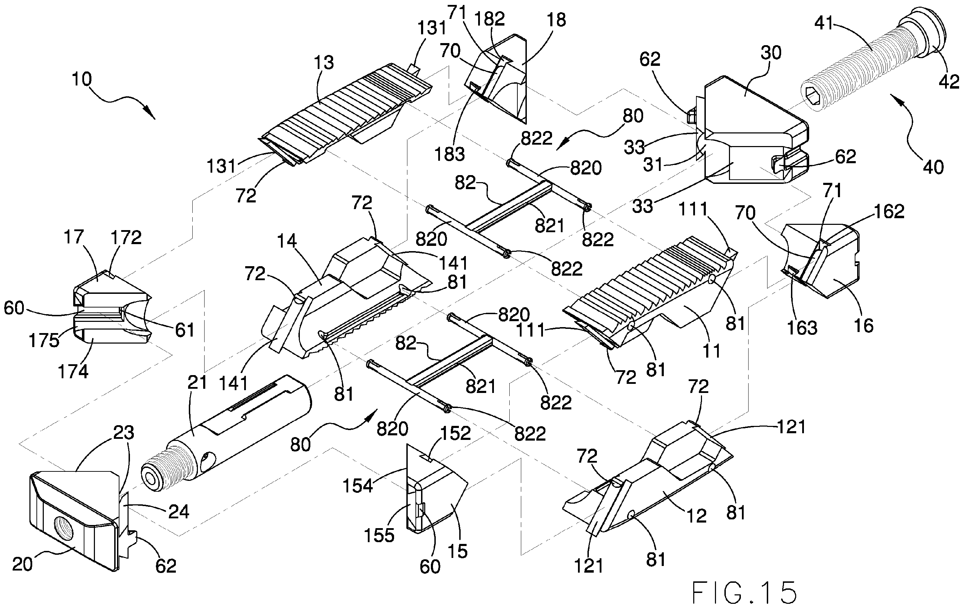

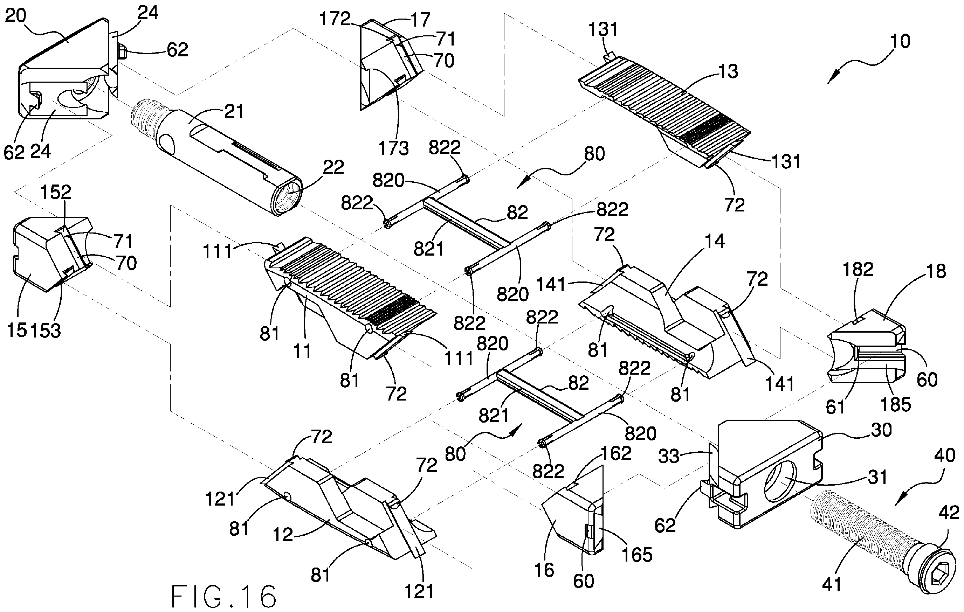

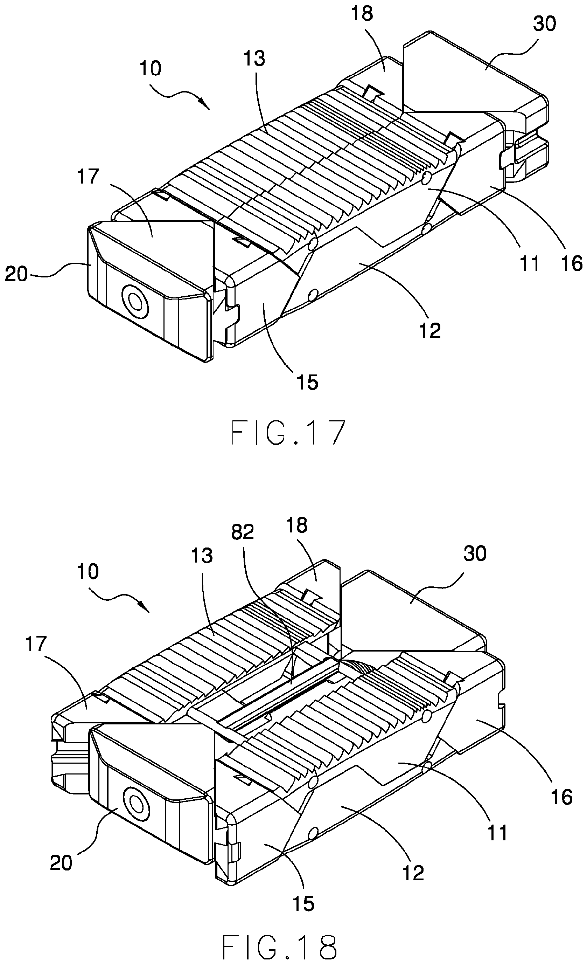

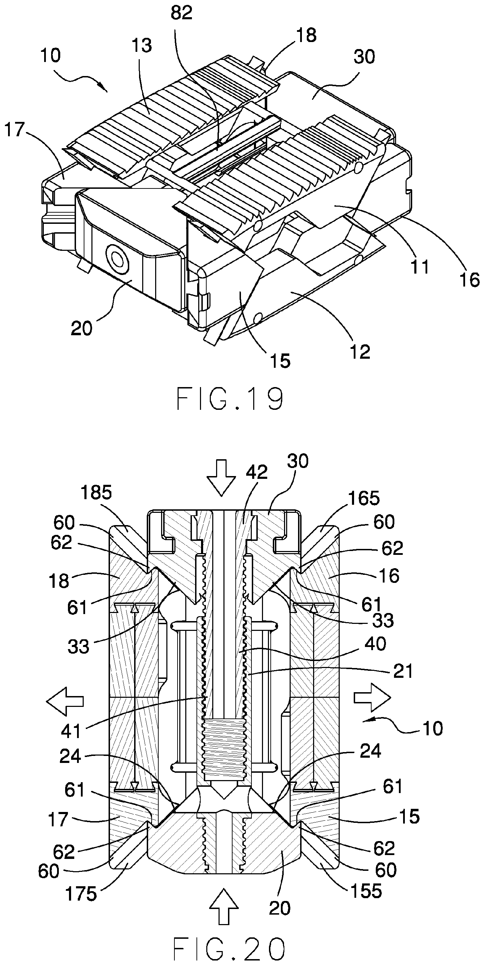

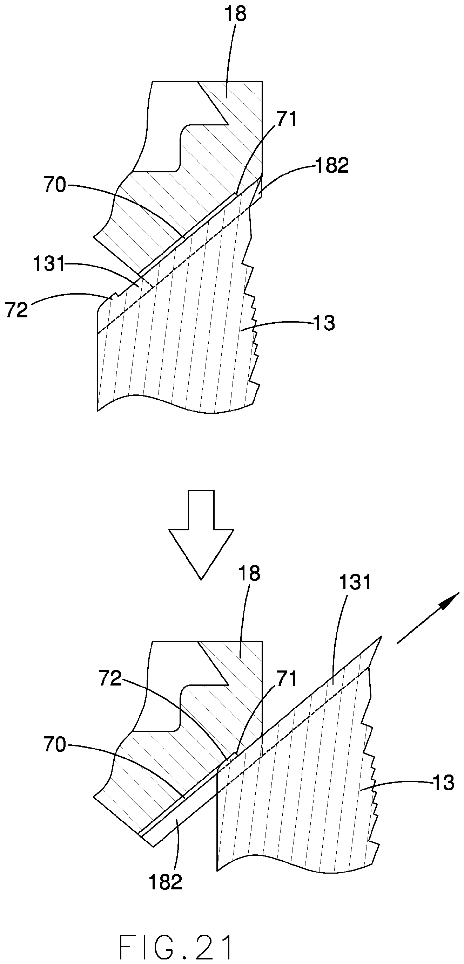

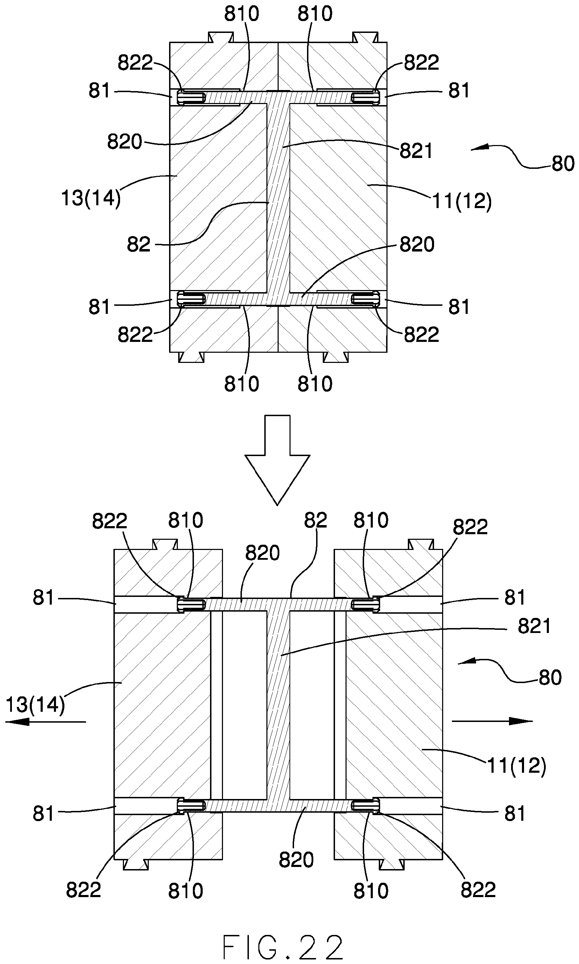

FIGS. 15-22 illustrate a second embodiment of the multi-section expandable device of the present disclosure. The second embodiment is substantially similar to the first embodiment with the exceptions described hereinafter.
(1) In the second embodiment, one end of the rod 21 is coupled to the first push member 20 in a screwed manner.
(2) In the first embodiment, the two first stop faces 25 and the two second stop faces 34 are respectively blocked by the two first front stop pieces 156, the two third front stop pieces 176 and the two second rear stop pieces 166, the two fourth rear stop pieces 86 as the extremity of the first-stage expansion, thus forming a condition for performing the second-stage expansion. In the second embodiment, each of the first front dovetailed groove 155, the second rear dovetailed groove 165, the third front dovetailed groove 175 and the fourth rear dovetailed groove 185 has an ultimate guide groove 60 on a bottom thereof. One end of the ultimate guide groove 60 is an open end, and another end of the ultimate guide groove 60 is formed with an ultimate stop face 61. One end of each of the two first push member guide rails 24 and the two second push member guide rails 33 is provided with a raised portion 62 to be inserted in the ultimate guide groove 60. Referring to FIG. 18 and FIG. 20, when the bolt 40 is tightened until the four raised portions 62 are respectively blocked by the ultimate stop faces 61 of the four ultimate guide grooves 60, the first and second expansion blocks 11, 12 and the third and fourth expansion blocks 13, 14 between the first and second push members 20, 30 cannot continue to expand laterally. At this time, the expansion module 10 is laterally expanded to have the maximum width (this is, the ultimate width of the first-stage expansion). Then, the bolt 40 is further tightened, and the first and second expansion blocks 11, 12 are moved along the first upper inclined plane 150 and the first lower inclined plane 151 of the first guide block 15 and the second upper inclined plane 160 and the second lower inclined plane 161 of the second guide block 16 to expand longitudinally. At the same time, the third and fourth expansion blocks 13, 14 are also moved along the third upper inclined plane 170 and the third lower inclined plane 171 of the third guide block 17 and the fourth upper inclined plane 180 and the fourth lower inclined plane 181 of the fourth guide block 18 to expand longitudinally.
(3) In the second embodiment, each of the first upper dovetailed groove 152, the first lower dovetailed groove 153, the second upper dovetailed groove 162, the second lower dovetailed groove 163, the third upper dovetailed groove 172, the third lower dovetailed groove 173, the fourth upper dovetailed groove 182 and the fourth lower dovetailed groove 183 is provided with a limit guide groove 170 on a bottom thereof. One end of the limit guide groove 70 is an open end, and another end of the limit guide groove 70 is formed with an engaging face 71. One end of each of the two first inclined plane guide rails 111, the two second inclined plane guide rails 121, the two third inclined plane guide rails 131 and the two fourth inclined plane guide rails 141 is provided with an engaging portion 72 to be inserted in the limit guide groove 70. The eight engaging portions 72 are blocked by the engaging faces 71 of the eight limit guide grooves 70 to prevent the first, second, third and fourth expansion blocks 11, 12, 13, 14 from being disengaged from the first, second, third and fourth guide blocks 15, 16, 17, 18 when they are expanded longitudinally. (FIG. 21 illustrates the limit operation of the third inclined plane guide rail 131 and the fourth upper dovetailed groove 182, and the other relative positions are the same. Therefore, it is no longer shown in the figures).
(4) In the second embodiment, two guide structures 80 are disposed between the first and third expansion blocks 11, 13 and between the second and fourth expansion blocks 12, 14, respectively. The two guide structures 80 facilitate the first and third expansion blocks 11, 13 and the second and fourth expansion blocks 12, 14 to move synchronously when they are expanded longitudinally. Referring to FIG. 22, the two guide structures 80 include a plurality of guide holes 81 that are transversely formed in the first, second, third and fourth expansion blocks 11, 12, 13, 14 (in this embodiment, two guide holes are taken as an example) and two guide members 82. The inner ends of the plurality of guide holes 81 each have a flange 810 with a smaller inner diameter. The two guide members 82 are disposed between the first and third expansion blocks 11, 13 and between the second and fourth expansion blocks 12, 14, respectively. Each of the guide members 82 includes two parallel guide rods 820 and a connecting rod 821 connected between the central portions of the two guide rods 820. Respective two ends of the two guide rods 820 of the two guide members 82 are telescopically inserted into the plurality of guide holes 81. The two ends of each guide rod 820 have two enlarged heads 822 with larger outer diameters that can be elastically retracted. When the guide rods 820 are inserted in the guide holes 81, the enlarged heads 822 are blocked by the flanges 810 of the guide holes 81 to prevent the guide rods 820 from being easily detached from the guide holes 81. Thus, when expanded laterally, the first and third expansion blocks 11, 13 and the second and fourth expansion blocks 12, 14 can be smoothly moved through the two guide structures 80, respectively. When expanded longitudinally, the first and third expansion blocks 11, 13 and the second and fourth expansion blocks 12, 14 can be synchronously moved up and down through the two guide structures 80, respectively.

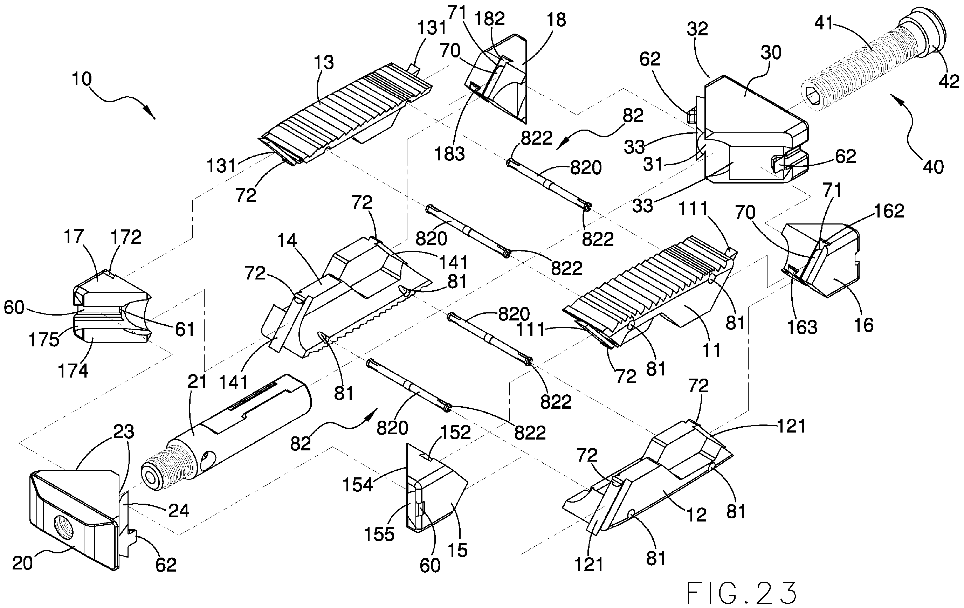

FIG. 23 illustrates a third embodiment of the multi-section expandable device of the present disclosure. The third embodiment is substantially similar to the second embodiment with the exceptions described hereinafter. In the third embodiment, the two guide members 82 each include two guide rods 820, without the connecting rod 821. In the third embodiment, the two guide members 82 each may include one, three or more guide rods 820. Because it is only an increase or decrease in number, the details will not be repeated.

FIGS. 24-26 illustrate a fourth embodiment of the multi-section expandable device of the present invention. The fourth embodiment is substantially similar to the third embodiment with the exceptions described hereinafter. In the fourth embodiment, the first expansion block 11, the second expansion block 12, the third expansion block 13 and the fourth expansion block 14 each comprise a front block a and a rear block b. The front block a and the rear block b are slidably connected to each other with corresponding inclined connecting faces a1, b1. The corresponding inclined connecting faces a1, b1 are provided with a dovetailed groove b2 and a guide rail a2 to be engaged with each other. The plurality of front blocks a and the plurality of rear blocks b can be expanded longitudinally to mate uneven opposite faces of the upper and lower vertebrae 50, 51 to form the second-stage expansion, so that the upper and lower sides of the first expansion block 11, the second expansion block 12, the third expansion block 13 and the fourth expansion block 14 can match the shape, height or angle of the opposite faces of the upper and lower vertebrae 50, 51 to make an adjustment so as to obtain good and stable support, which can be applied to patients with different conditions.

In the present invention, the first upper dovetailed groove 152 and the first inclined plane guide rail 111, the first lower dovetailed groove 153 and the second inclined plane guide rail 121, the second upper dovetailed groove 162 and the first inclined plane guide rail 111, the second lower dovetailed groove 163 and the second inclined plane guide rail 121, the third upper dovetailed groove 172 and the third inclined plane guide rail 131, the third lower dovetailed groove 173 and the fourth inclined plane guide rail 141, the fourth upper dovetailed groove 182 and the third inclined plane guide rail 131, the fourth lower dovetailed groove 183 and the fourth inclined plane guide rail 141, the first and third front dovetailed grooves 155, 175 and the two first push member guide rails 24, the second and fourth rear dovetailed grooves 165, 185 and the two second push member guide rails 33, the dovetailed groove b2 and the guide rail a2 may be other concave and convex engaging structures that can be slidably engaged with each other.

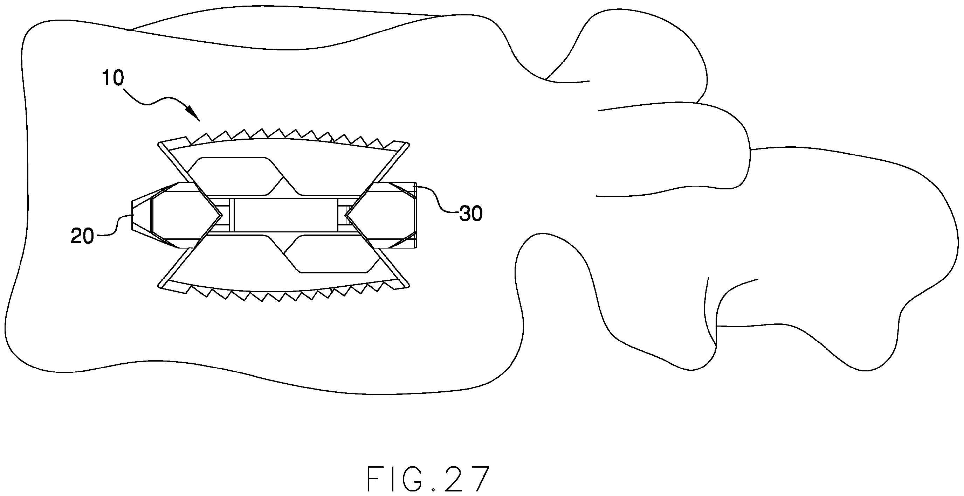

Referring to FIG. 27, the multi-section expandable device of the present invention may be applied to bones (such as, inside the vertebra of the spine) for the restoration or repair of the depressed fracture. The multi-section expandable device of the present invention may also be applied to other related parts such as a space between intervertebral space, bones or soft tissues (such as restoration or repair of face depression). The material of the multi-section expandable device may be changed depending on the location of the application, for example, it may be metal, polymer material, PE, silicone, etc. The multi-section expandable device can be implanted horizontally or vertically to achieve the above-mentioned functions and scope of application.

Although particular embodiments of the present invention have been described in detail for purposes of illustration, various modifications and enhancements may be made without departing from the spirit and scope of the present invention. Accordingly, the present invention is not to be limited except as by the appended claims.

## Claims

1. A multi-section expandable device, comprising:
an expansion module (10), able to expand longitudinally and laterally;
a first push member (20), disposed at a front end of the expansion module (10) and having a screw hole (22) therein;
a second push member (30), disposed at a rear end of the expansion module (10) and having a through hole (31) therein; and
a bolt (40), having a screw portion (41) and a head portion (42), the screw portion (41) being inserted through the through hole (31) of the second push member (30) and screwed in the screw hole (22) of the first push member (20), the head portion (42) abutting against one side of the second push member (30) opposite to the first push member (20),
wherein when the bolt (40) is tightened, the first push member (20) and the second push member (30) are pushed to approach each other so as to push the expansion module (10) to generate a first-stage expansion and a second-stage expansion, the first-stage expansion enables the expansion module (10) to expand laterally so as to adjust its width, the second-stage expansion is performed after the expansion module (10) is laterally expanded to have a maximum width, so that the expansion module (10) is longitudinally expanded to adjust its height;
wherein the expansion module (10) includes a first expansion block (11), a second expansion block (12), a third expansion block (13), a fourth expansion block (14), a first guide block (15), a second guide block (16), a third guide block (17), and a fourth guide block (18); the second expansion block (12) is located under the first expansion block (11), the third expansion block (13) is located beside the first expansion block (11), the fourth expansion block (14) is located under the third expansion block (13); the first guide block (15) is slidably disposed on front end faces of the first and second expansion blocks (11, 12), the second guide block (16) is slidably disposed on rear end faces of the first and second expansion blocks (11, 12), the first and second expansion blocks (11, 12) are moved up and down between the first and second guide blocks (15, 16) to expand longitudinally; the third guide block (17) is slidably disposed on front end faces of the third and fourth expansion blocks (13, 14), the fourth guide block (18) is slidably disposed on rear end faces of the third and fourth expansion blocks (13, 14), the third and fourth expansion blocks (13, 14) are moved up and down between the third and fourth guide blocks (17, 18) to expand longitudinally; the first push member (20) is slidably disposed on front end faces of the first and third guide blocks (15, 17), the second push member (30) is slidably disposed on rear end faces of the second and fourth guide blocks (16, 18), the first and second guide blocks (15, 16) and the third and fourth guide blocks (17, 18) respectively drive the first and second expansion blocks (11, 12) and the third and fourth expansion blocks (13, 14) to move left and right between the first and second push members (20, 30) so as to expand laterally;
wherein the first expansion block (11), the second expansion block (12), the third expansion block (13) and the fourth expansion block (14) each comprise a front block (a) and a rear block (b), the front block (a) and the rear block (b) are slidably connected to each other with corresponding inclined connecting faces (a1, b1), the front blocks (a) and the rear blocks (b) of the first, second, third and the fourth expansion blocks (11, 12, 13, 14) can be expanded longitudinally to mate uneven opposite faces of upper and lower vertebrae (50, 51) to form the second-stage expansion;
wherein the corresponding inclined connecting faces (a1, b1) are provided with a dovetailed groove (b2) and a guide rail (a2) to be engaged with each other.

2. The multi-section expandable device as claimed in claim 1, wherein
front and rear ends of the first expansion block (11) are formed with two first inclined planes (110) that are inclined outwardly from bottom to top, respectively;
front and rear ends of the second expansion block (12) are formed with two second inclined planes (120) that are inclined outwardly from top to bottom, respectively;
front and rear ends of the third expansion block (13) are formed with two third inclined planes (130) that are inclined outwardly from bottom to top, respectively;
front and rear ends of the fourth expansion block (14) are formed with two fourth inclined planes (140) that are inclined outwardly from top to bottom, respectively;
a rear end of the first guide block (15) is formed with a first upper inclined plane (150) and a first lower inclined plane (151) corresponding to the first and second inclined planes (110, 120) on the front ends of the first and second expansion blocks (11, 12), a front end of the first guide block (15) is formed with a first front inclined plane (154) that is inclined outwardly from back to front;
a front end of the second guide block (16) is formed with a second upper inclined plane (160) and a second lower inclined plane (161) corresponding to the first and second inclined planes (110, 120) on the rear ends of the first and second expansion blocks (11, 12), a rear end of the second guide block (16) is formed with a second rear inclined plane (164) that is inclined outwardly from front to back;
a rear end of the third guide block (17) is formed with a third upper inclined plane (170) and a third lower inclined plane (171) corresponding to the third and fourth inclined planes (130, 140) on the front ends of the third and fourth expansion blocks (13, 14), a front end of the third guide block (17) is formed with a third front inclined plane (174) that is inclined outwardly from back to front;
a front end of the fourth guide block (18) is formed with a fourth upper inclined plane (180) and a fourth lower inclined plane (181) corresponding to the third and fourth inclined planes (130, 140) on the rear ends of the third and fourth expansion blocks (13, 14), a rear end of the fourth guide block (18) is formed with a fourth rear inclined plane (184) that is inclined outwardly from front to back;
two sides of the first push member (20) have two first push member inclined planes (23) corresponding to the first and third front inclined planes (154, 174);
two sides of the second push member (30) have two second push member inclined planes (32) corresponding to the second and fourth rear inclined planes (164, 184);
concave and convex engaging structures that can be slidably engaged with each other are disposed between the two first inclined planes (110) and the first and second upper inclined planes (150, 160), between the two second inclined planes (120) and the first and second lower inclined planes (151, 161), between the two third inclined planes (130) and the third and fourth upper inclined planes (170, 180), between the two fourth inclined planes (140) and the third and fourth lower inclined planes (171, 181), between the two first push member inclined planes (23) and the first and third front inclined planes (154, 174), and between the two second push member inclined plane (32) and the second and fourth rear inclined planes (164, 184), respectively.

3. The multi-section expandable device as claimed in claim 2, wherein two first inclined plane guide rails (111) are disposed on the two first inclined planes (110), respectively; two second inclined plane guide rails (121) are disposed on the two second inclined planes (120), respectively; two second inclined plane guide rails (113) are disposed on the two second inclined planes (130), respectively; two fourth inclined plane guide rails (141) are disposed on the two fourth inclined planes (140), respectively; the first upper inclined plane (150) is provided with a first upper dovetailed groove (152) for the first inclined plane guide rail (111) on the front end of the first expansion block (11) to be inserted therein, the first lower inclined plane (151) is provided with a first lower dovetailed groove (153) for the second inclined plane guide rail (121) on the front end of the second expansion block (12) to be inserted therein, the first front inclined plane (154) is provided with a first front dovetailed groove (155); the second upper inclined plane (160) is provided with a second upper dovetailed groove (162) for the first inclined plane guide rail (111) on the rear end of the first expansion block (11) to be inserted therein, the second lower inclined plane (161) is provided with a second lower dovetailed groove (163) for the second inclined plane guide rail (121) on the rear end of the second expansion block (12) to be inserted therein, the second rear inclined plane (164) is provided with a second rear dovetailed groove (165); the third upper inclined plane (170) is provided with a third upper dovetailed groove (172) for the third inclined plane guide rail (131) on the front end of the third expansion block (13) to be inserted therein, the third lower inclined plane (171) is provided with a third lower dovetailed groove (173) for the fourth inclined plane guide rail (141) on the front end of the fourth expansion block (14) to be inserted therein, the third front inclined plane (174) is provided with a third front dovetailed groove (175); the fourth upper inclined plane (180) is provided with a fourth upper dovetailed groove (182) for the third inclined plane guide rail (131) on the rear end of the third expansion block (13) to be inserted therein, the fourth lower inclined plane (181) is provided with a fourth lower dovetailed groove (183) for the fourth inclined plane guide rail (141) on the rear end of the fourth expansion block (14) to be inserted therein, the fourth front inclined plane (184) is provided with a fourth rear dovetailed groove (185); the two first push member inclined planes (23) are provided with two first push member guide rails (24) to be inserted in the first and third front dovetailed grooves (155, 175); and the two second push member inclined planes (32) are provided with two second push member guide rails (33) to be inserted in the second and fourth rear dovetailed grooves (165, 185).

4. The multi-section expandable device as claimed in claim 3, wherein two first front stop pieces (156) are protruded from a lower end of the first front inclined plane (154), two second rear stop pieces (166) are protruded from a lower end of the second rear inclined plane (164), two third front stop pieces (176) are protruded from a lower end of the third front inclined plane (174), two fourth rear stop pieces (186) are protruded from a lower end of the fourth rear inclined plane (184); the first push member (20) has two first stop faces (25) on upper and lower sides thereof, the second push member (30) has two second stop faces (34) on upper and lower sides thereof; when the two first stop faces (25) and the two second stop faces (34) are respectively blocked by the two first front stop pieces (156), the two third front stop pieces (176) and the two second rear stop pieces (166), the two four rear stop pieces (186), the first and second expansion blocks (11, 12) and the third and fourth expansion blocks (13, 14) cannot continue to expand laterally, thereby achieving an ultimate width of the first-stage expansion.

5. The multi-section expandable device as claimed in claim 1, wherein a rod (21) is protruded from a rear side of the first push member (20), and the screw hole (22) is disposed in the rod (21).

6. The multi-section expandable device as claimed in claim 5, wherein one end of the rod (21) is coupled to the first push member (20) in a screwed manner.

7. The multi-section expandable device as claimed in claim 1, wherein a bottom surface of the first expansion block (11) has a plurality of first guide protrusions (112) and a plurality of first guide grooves (113) which are staggered and each have a trapezoidal shape; a top surface of the second expansion block (12) has a plurality of second guide grooves (122) and a plurality of second guide protrusions (123) which can be slid up and down relative to the plurality of first guide protrusions (112) and the plurality of first guide grooves (113); a bottom surface of the third expansion block (13) has a plurality of third guide protrusions (132) and a plurality of third guide grooves (133) which are staggered and each have a trapezoidal shape; a top surface of the fourth expansion block (14) has a plurality of fourth guide grooves (142) and a plurality of fourth guide protrusions (143) which can be slid up and down relative to the plurality of third guide protrusions (132) and the plurality of third guide grooves (133).

8. The multi-section expandable device as claimed in claim 3, wherein each of the first front dovetailed groove (155), the second rear dovetailed groove (165), the third front dovetailed groove (175) and the fourth rear dovetailed groove (185) has an ultimate guide groove (60) on a bottom thereof, one end of the ultimate guide groove (60) is an open end, another end of the ultimate guide groove (60) is formed with an ultimate stop face (61), one end of each of the two first push member guide rails (24) and the two second push member guide rails (33) is provided with a raised portion (62) to be inserted in the ultimate guide groove (60), when the four raised portions (62) are respectively blocked by the ultimate stop faces (61) of the four ultimate guide grooves (60), the first and second expansion blocks (11, 12) and the third and fourth expansion blocks (13, 14) cannot continue to expand laterally, thereby achieving an ultimate width of the first-stage expansion.

9. The multi-section expandable device as claimed in claim 4 or 8, wherein each of the first upper dovetailed groove (152), the first lower dovetailed groove (153), the second upper dovetailed groove (162), the second lower dovetailed groove (163), the third upper dovetailed groove (172), the third lower dovetailed groove (173), the fourth upper dovetailed groove (182) and the fourth lower dovetailed groove (183) is provided with a limit guide groove (170) on a bottom thereof, one end of the limit guide groove (70) is an open end, another end of the limit guide groove (70) is formed with an engaging face (71); one end of each of the two first inclined plane guide rails (111), the two second inclined plane guide rails (121), the two third inclined plane guide rails (131) and the two fourth inclined plane guide rails (141) is provided with an engaging portion (72) to be inserted in the limit guide groove (70); the eight engaging portions (72) are blocked by the engaging faces (71) of the eight limit guide grooves (70) to prevent the first, second, third and fourth expansion blocks (11, 12, 13, 14) from being disengaged from the first, second, third and fourth guide blocks (15, 16, 17, 18).

10. The multi-section expandable device as claimed in claim 1, further comprising two guide structures (80), the two guide structures (80) being disposed between the first and third expansion blocks (11, 13) and between the second and fourth expansion blocks (12, 14), respectively; the two guide structures (80) facilitate the first and third expansion blocks (11, 13) and the second and fourth expansion blocks (12, 14) to move synchronously when expanded.

11. The multi-section expandable device as claimed in claim 10, wherein the two guide structures (80) include a plurality of guide holes (81) that are transversely formed in the first, second, third and fourth expansion blocks (11, 12, 13, 14) and two guide members (82), the two guide members (82) are respectively disposed between the first and third expansion blocks (11, 13) and between the second and fourth expansion blocks (12, 14), each of the guide members (82) includes at least one guide rod (820), and two ends of the guide rod (820) are telescopically inserted into corresponding two of the guide holes (81).

12. The multi-section expandable device as claimed in claim 10, wherein the two guide structures (80) include a plurality of guide holes (81) that are transversely formed in the first, second, third and fourth expansion blocks (11, 12, 13, 14) and two guide members (82), the two guide members (82) are respectively disposed between the first and third expansion blocks (11, 13) and between the second and fourth expansion blocks (12, 14), each of the guide members (82) includes two parallel guide rods (820) and a connecting rod (821) connected between central portions of the two guide rods (820), and respective two ends of the two guide rods (820) are telescopically inserted into the plurality of guide holes (81).

## Patentansprüche

1. Expandierbare Vorrichtung mit mehreren Abschnitten, umfassend:
ein Expansionsmodul (10), das in der Lage ist, sich in Längsrichtung und seitlich zu expandieren;
ein erstes Schubelement (20), das an einem vorderen Ende des Expansionsmoduls (10) angeordnet ist und ein Schraubenloch (22) darin aufweist;
ein zweites Schubelement (30), das an einem hinteren Ende des Expansionsmoduls (10) angeordnet ist und ein Durchgangsloch (31) darin aufweist; und
einen Bolzen (40), der einen Schraubenabschnitt (41) und einen Kopfabschnitt (42) aufweist, wobei der Schraubenabschnitt (41) durch das Durchgangsloch (31) des zweiten Schubelements (30) eingeführt und in das Schraubenloch (22) des ersten Schubelements (20) geschraubt ist, wobei der Kopfabschnitt (42) an einer Seite des zweiten Schubelements (30) gegenüber dem ersten Schubelement (20) anliegt,
wobei, wenn der Bolzen (40) angezogen wird, das erste Schubelement (20) und das zweite Schubelement (30) so geschoben werden, dass sie sich einander annähern, um das Expansionsmodul (10) so zu schieben, dass eine Expansion einer ersten Stufe und eine Expansion einer zweiten Stufe erzeugt wird, wobei die Expansion der ersten Stufe es dem Expansionsmodul (10) ermöglicht, sich seitlich zu expandieren, um seine Breite einzustellen, wobei die Expansion der zweiten Stufe durchgeführt wird, nachdem das Expansionsmodul (10) seitlich so expandiert wurde, dass es eine maximale Breite aufweist, so dass das Expansionsmodul (10) in Längsrichtung expandiert wird, um seine Höhe einzustellen;
wobei das Expansionsmodul (10) einen ersten Expansionsblock (11), einen zweiten Expansionsblock (12), einen dritten Expansionsblock (13), einen vierten Expansionsblock (14), einen ersten Führungsblock (15), einen zweiten Führungsblock (16), einen dritten Führungsblock (17) und einen vierten Führungsblock (18) umfasst;
der zweite Expansionsblock (12) unter dem ersten Expansionsblock (11) angeordnet ist, der dritte Expansionsblock (13) neben dem ersten Expansionsblock (11) angeordnet ist, der vierte Expansionsblock (14) unter dem dritten Expansionsblock (13) angeordnet ist; der erste Führungsblock (15) verschiebbar an vorderen Endflächen des ersten und des zweiten Expansionsblocks (11, 12) angeordnet ist, der zweite Führungsblock (16) verschiebbar an hinteren Endflächen des ersten und des zweiten Expansionsblocks (11, 12) angeordnet ist, der erste und der zweite Expansionsblock (11, 12) zwischen dem ersten und dem zweiten Führungsblock (15, 16) auf und ab bewegt werden, um sich in Längsrichtung zu expandieren;
der dritte Führungsblock (17) verschiebbar an vorderen Endflächen des dritten und des vierten Expansionsblocks (13, 14) angeordnet ist, der vierte Führungsblock (18) verschiebbar an hinteren Endflächen des dritten und des vierten Expansionsblocks (13, 14) angeordnet ist, der dritte und der vierte Expansionsblock (13, 14) zwischen dem dritten und dem vierten Führungsblock (17, 18) auf und ab bewegt werden, um in Längsrichtung zu expandieren; das erste Schubelement (20) verschiebbar an vorderen Endflächen des ersten und des dritten Führungsblocks (15, 17) angeordnet ist, das zweite Schubelement (30) verschiebbar an hinteren Endflächen des zweiten und des vierten Führungsblocks (16, 18) angeordnet ist, der erste und der zweite Führungsblock (15, 16) und der dritte und der vierte Führungsblock (17, 18) den ersten bzw. den zweiten Expansionsblock (11, 12) und den dritten bzw. den vierten Expansionsblock (13, 14) so antreiben, dass sie sich zwischen dem ersten und dem zweiten Schubelement (20, 30) nach links und rechts bewegen, um seitlich zu expandieren;
wobei der erste Expansionsblock (11), der zweite Expansionsblock (12), der dritte Expansionsblock (13) und der vierte Expansionsblock (14) jeweils einen vorderen Block (a) und einen hinteren Block (b) umfassen, der vordere Block (a) und der hintere Block (b) mit entsprechenden geneigten Verbindungsflächen (a1, b1) verschiebbar miteinander verbunden sind, die vorderen Blöcke (a) und die hinteren Blöcke (b) des ersten, des zweiten, des dritten und des vierten Expansionsblocks (11, 12, 13, 14) in Längsrichtung expandiert werden können, um mit unebenen gegenüberliegenden Flächen des oberen und des unteren Wirbels (50, 51) zusammenzupassen, um die Expansion der zweiten Stufe zu bilden;
wobei die entsprechenden geneigten Verbindungsflächen (a1, b1) mit einer Schwalbenschwanznut (b2) und einer Führungsschiene (a2) versehen sind, um miteinander in Eingriff gebracht zu werden.

2. Expandierbare Vorrichtung mit mehreren Abschnitten nach Anspruch 1, wobei
das vordere und das hintere Ende des ersten Expansionsblocks (11) mit zwei ersten geneigten Ebenen (110) gebildet sind, die jeweils von unten nach oben nach außen geneigt sind;
das vordere und das hintere Ende des zweiten Expansionsblocks (12) mit zwei zweiten geneigten Ebenen (120) gebildet sind, die jeweils von oben nach unten nach außen geneigt sind;
das vordere und das hintere Ende des dritten Expansionsblocks (13) mit zwei dritten geneigten Ebenen (130) gebildet sind, die jeweils von unten nach oben nach außen geneigt sind;
das vordere und das hintere Ende des vierten Expansionsblocks (14) mit zwei vierten geneigten Ebenen (140) gebildet sind, die jeweils von oben nach unten nach außen geneigt sind;
ein hinteres Ende des ersten Führungsblocks (15) mit einer ersten oberen geneigten Ebene (150) und einer ersten unteren geneigten Ebene (151) gebildet ist, die der ersten und der zweiten geneigten Ebene (110, 120) an den vorderen Enden des ersten und des zweiten Expansionsblocks (11, 12) entsprechen, ein vorderes Ende des ersten Führungsblocks (15) mit einer ersten vorderen geneigten Ebene (154) gebildet ist, die von hinten nach vorne nach außen geneigt ist;
ein vorderes Ende des zweiten Führungsblocks (16) mit einer zweiten oberen geneigten Ebene (160) und einer zweiten unteren geneigten Ebene (161) gebildet ist, die der ersten und der zweiten geneigten Ebene (110, 120) an den hinteren Enden des ersten und des zweiten Expansionsblocks (11, 12) entsprechen, ein hinteres Ende des zweiten Führungsblocks (16) mit einer zweiten hinteren geneigten Ebene (164) gebildet ist, die von vorne nach hinten nach außen geneigt ist;
ein hinteres Ende des dritten Führungsblocks (17) mit einer dritten oberen geneigten Ebene (170) und einer dritten unteren geneigten Ebene (171) gebildet ist, die der dritten und der vierten geneigten Ebene (130, 140) an den vorderen Enden des dritten und des vierten Expansionsblocks (13, 14) entsprechen, ein vorderes Ende des dritten Führungsblocks (17) mit einer dritten vorderen geneigten Ebene (174) gebildet ist, die von hinten nach vorne nach außen geneigt ist;
ein vorderes Ende des vierten Führungsblocks (18) mit einer vierten oberen geneigten Ebene (180) und einer vierten unteren geneigten Ebene (181) gebildet ist, die der dritten und der vierten geneigten Ebene (130, 140) an den hinteren Enden des dritten und des vierten Expansionsblocks (13, 14) entsprechen, ein hinteres Ende des vierten Führungsblocks (18) mit einer vierten hinteren geneigten Ebene (184) gebildet ist, die von vorne nach hinten nach außen geneigt ist;
zwei Seiten des ersten Schubelements (20) zwei erste Schubelement-Neigungsebenen (23) aufweisen, die der ersten und der dritten vorderen geneigten Ebene (154, 174) entsprechen; zwei Seiten des zweiten Schubelements (30) zwei zweite Schubelement-Neigungsebenen (32) aufweisen, die der zweiten und der vierten hinteren geneigten Ebene (164, 184) entsprechen; konkave und konvexe Eingriffsstrukturen, die gleitend miteinander in Eingriff gebracht werden können, zwischen den zwei ersten geneigten Ebenen (110) und der ersten und der zweiten oberen geneigten Ebene (150, 160), zwischen den zwei zweiten geneigten Ebenen (120) und der ersten und der zweiten unteren geneigten Ebene (151, 161), zwischen den zwei dritten geneigten Ebenen (130) und der dritten und der vierten oberen geneigten Ebene (170, 180), zwischen den zwei vierten geneigten Ebenen (140) und der dritten und der vierten unteren geneigten Ebene (171, 181), zwischen den zwei ersten Schubelement-Neigungsebenen (23) und der ersten und der dritten vorderen geneigten Ebene (154, 174) bzw. zwischen den zwei zweiten Schubelement-Neigungsebenen (32) und der zweiten und der vierten hinteren geneigten Ebene (164, 184) angeordnet sind.

3. Expandierbare Vorrichtung mit mehreren Abschnitten nach Anspruch 2, wobei zwei Führungsschienen (111) der ersten geneigten Ebene an den zwei ersten geneigten Ebenen (110) angeordnet sind;
zwei Führungsschienen (121) der zweiten geneigten Ebene an den zwei zweiten geneigten Ebenen (120) angeordnet sind; zwei Führungsschienen (113) der zweiten geneigten Ebene an den zwei zweiten geneigten Ebenen (130) angeordnet sind; zwei Führungsschienen (141) der vierten geneigten Ebene an den zwei vierten geneigten Ebenen (140) angeordnet sind; die erste obere geneigte Ebene (150) mit einer ersten oberen Schwalbenschwanznut (152) versehen ist, damit die Führungsschiene (111) der ersten geneigten Ebene am vorderen Ende des ersten Expansionsblocks (11) darin eingeführt werden kann, die erste untere geneigte Ebene (151) mit einer ersten unteren Schwalbenschwanznut (153) versehen ist, damit die Führungsschiene (121) der zweiten geneigten Ebene am vorderen Ende des zweiten Expansionsblocks (12) darin eingeführt werden kann, die erste vordere geneigte Ebene (154) mit einer ersten vorderen Schwalbenschwanznut (155) versehen ist; die zweite obere geneigte Ebene (160) mit einer zweiten oberen Schwalbenschwanznut (162) versehen ist, damit die Führungsschiene (111) der ersten geneigten Ebene am hinteren Ende des ersten Expansionsblocks (11) darin eingeführt werden kann, die zweite untere geneigte Ebene (161) mit einer zweiten unteren Schwalbenschwanznut (163) versehen ist, damit die Führungsschiene (121) der zweiten geneigten Ebene am hinteren Ende des zweiten Expansionsblocks (12) darin eingeführt werden kann, die zweite hintere geneigte Ebene (164) mit einer zweiten hinteren Schwalbenschwanznut (165) versehen ist; die dritte obere geneigte Ebene (170) mit einer dritten oberen Schwalbenschwanznut (172) versehen ist, damit die Führungsschiene (131) der dritten geneigten Ebene am vorderen Ende des dritten Expansionsblocks (13) darin eingeführt werden kann, die dritte untere geneigte Ebene (171) mit einer dritten unteren Schwalbenschwanznut (173) versehen ist, damit die Führungsschiene (141) der vierten geneigten Ebene am vorderen Ende des vierten Expansionsblocks (14) darin eingeführt werden kann, die dritte vordere geneigte Ebene (174) mit einer dritten vorderen Schwalbenschwanznut (175) versehen ist; die vierte obere geneigte Ebene (180) mit einer vierten oberen Schwalbenschwanznut (182) versehen ist, damit die Führungsschiene (131) der dritten geneigten Ebene am hinteren Ende des dritten Expansionsblocks (13) darin eingeführt werden kann, die vierte untere geneigte Ebene (181) mit einer vierten unteren Schwalbenschwanznut (183) versehen ist, damit die Führungsschiene (141) der vierten geneigten Ebene am hinteren Ende des vierten Expansionsblocks (14) darin eingeführt werden kann, die vierte vordere geneigte Ebene (184) mit einer vierten hinteren Schwalbenschwanznut (185) versehen ist;
die zwei ersten Schubelement-Neigungsebenen (23) mit zwei ersten Schubelement-Führungsschienen (24) versehen sind, um in die erste und die dritte vordere Schwalbenschwanznut (155, 175) eingeführt zu werden; und die zwei zweiten Schubelement-Neigungsebenen (32) mit zwei zweiten Schubelement-Führungsschienen (33) versehen sind, um in die zweite und die vierte hintere Schwalbenschwanznut (165, 185) eingeführt zu werden.

4. Expandierbare Vorrichtung mit mehreren Abschnitten nach Anspruch 3, wobei zwei erste vordere Anschläge
zwei zweite vordere Anschläge (156) von einem unteren Ende der ersten vorderen geneigten Ebene (154) vorstehen, zwei zweite hintere Anschläge (166) von einem unteren Ende der zweiten hinteren geneigten Ebene (164) vorstehen, zwei dritte vordere Anschläge (176) von einem unteren Ende der dritten vorderen geneigten Ebene (174) vorstehen, zwei vierte hintere Anschläge (186) von einem unteren Ende der vierten hinteren geneigten Ebene (184) vorstehen; das erste Schubelement (20) zwei erste Anschlagflächen (25) auf seiner oberen und seiner unteren Seite aufweist, das zweite Schubelement (30) zwei zweite Anschlagflächen (34) auf seiner oberen und seiner unteren Seite aufweist; wenn die zwei ersten Anschlagflächen (25) und die zwei zweiten Anschlagflächen (34) jeweils durch die zwei ersten vorderen Anschläge (156), die zwei dritten vorderen Anschläge (176) und die zwei zweiten hinteren Anschläge (166) blockiert werden, die zwei vierten hinteren Anschläge (186), der erste und der zweite Expansionsblock (11, 12) und der dritte und der vierte Expansionsblock (13, 14) nicht weiter seitlich expandieren können, wodurch eine endgültige Breite der Expansion der ersten Stufe erreicht wird.

5. Expandierbare Vorrichtung mit mehreren Abschnitten nach Anspruch 1, wobei eine Stange (21) von einer Rückseite des ersten Schubelements (20) vorsteht und das Schraubenloch (22) in der Stange (21) angeordnet ist.

6. Expandierbare Vorrichtung mit mehreren Abschnitten nach Anspruch 5, wobei ein Ende der Stange (21) mit dem ersten Schubelement (20) verschraubt ist.

7. Expandierbare Vorrichtung mit mehreren Abschnitten nach Anspruch 1, wobei eine untere Oberfläche des ersten Expansionsblocks (11) eine Vielzahl von ersten Führungsvorsprüngen (112) und eine Vielzahl von ersten Führungsnuten (113) aufweist, die versetzt sind und jeweils eine Trapezform aufweisen; eine obere Oberfläche des zweiten Expansionsblocks (12) eine Vielzahl von zweiten Führungsnuten (122) und eine Vielzahl von zweiten Führungsvorsprüngen (123) aufweist, die relativ zu der Vielzahl von ersten Führungsvorsprüngen (112) und der Vielzahl von ersten Führungsnuten (113) auf und ab geschoben werden können; eine untere Oberfläche des dritten Expansionsblocks (13) eine Vielzahl von dritten Führungsvorsprüngen (132) und eine Vielzahl von dritten Führungsnuten (133) aufweist, die versetzt sind und jeweils eine Trapezform aufweisen; eine obere Oberfläche des vierten Expansionsblocks (14) eine Vielzahl von vierten Führungsnuten (142) und eine Vielzahl von vierten Führungsvorsprüngen (143) aufweist, die relativ zu der Vielzahl von dritten Führungsvorsprüngen (132) und der Vielzahl von dritten Führungsnuten (133) auf und ab geschoben werden können.

8. Expandierbare Vorrichtung mit mehreren Abschnitten nach Anspruch 3, wobei jede der ersten vorderen Schwalbenschwanznut (155), der zweiten hinteren Schwalbenschwanznut (165), der dritten vorderen Schwalbenschwanznut (175) und der vierten hinteren Schwalbenschwanznut (185) eine abschließende Führungsnut (60) an einem Boden davon aufweist, ein Ende der abschließenden Führungsnut (60) ein offenes Ende ist, ein anderes Ende der abschließenden Führungsnut (60) mit einer abschließenden Anschlagfläche (61) ausgebildet ist, ein Ende jeder der zwei ersten Schubelement-Führungsschienen (24) und der zwei zweiten Schubelement-Führungsschienen (33) mit einem erhöhten Abschnitt (62) versehen ist, der in die abschließende Führungsnut (60) eingesetzt werden soll, wenn die vier erhöhten Abschnitte (62) jeweils durch die abschließenden Anschlagflächen (61) der vier abschließenden Führungsnuten (60) blockiert sind, der erste und der zweite Expansionsblock (11, 12) und der dritte und der vierte Expansionsblock (13, 14) nicht weiter lateral expandieren können, wodurch eine abschließende Breite der Expansion der ersten Stufe erreicht wird.

9. Expandierbare Vorrichtung mit mehreren Abschnitten nach Anspruch 4 oder 8, wobei jede der ersten oberen Schwalbenschwanznut (152), der ersten unteren Schwalbenschwanznut (153), der zweiten oberen Schwalbenschwanznut (162), der zweiten unteren Schwalbenschwanznut (163), der dritten oberen Schwalbenschwanznut (172), der dritten unteren Schwalbenschwanznut (173), der vierten oberen Schwalbenschwanznut (182) und der vierten unteren Schwalbenschwanznut (183) mit einer Begrenzungsführungsnut (170) an einem Boden davon versehen ist, ein Ende der Begrenzungsführungsnut (70) ein offenes Ende ist, ein anderes Ende der Begrenzungsführungsnut (70) mit einer Eingriffsfläche (71) ausgebildet ist; ein Ende jeder der zwei Führungsschienen (111) der ersten Neigungsebene, der zwei Führungsschienen (121) der zweiten Neigungsebene, der zwei Führungsschienen (131) der dritten Neigungsebene und der zwei Führungsschienen (141) der vierten Neigungsebene mit einem Eingriffsabschnitt (72) versehen ist, der in die Begrenzungsführungsnut (70) eingesetzt werden soll; die acht Eingriffsabschnitte (72) durch die Eingriffsflächen (71) der acht Begrenzungsführungsnuten (70) blockiert sind, um zu verhindern, dass der erste, der zweite, der dritte und der vierte Expansionsblock (11, 12, 13, 14) von dem ersten, dem zweiten, dem dritten und dem vierten Führungsblock (15, 16, 17, 18) gelöst werden.

10. Expandierbare Vorrichtung mit mehreren Abschnitten nach Anspruch 1, ferner umfassend zwei Führungsstrukturen (80), wobei die zwei Führungsstrukturen (80) zwischen dem ersten und dem dritten Expansionsblock (11, 13) bzw. zwischen dem zweiten und dem vierten Expansionsblock (12, 14) angeordnet sind;
die zwei Führungsstrukturen (80) ermöglichen, dass sich der erste und der dritte Expansionsblock (11, 13) und der zweite und der vierte Expansionsblock (12, 14) synchron bewegen, wenn sie expandiert werden.

11. Expandierbare Vorrichtung mit mehreren Abschnitten nach Anspruch 10, wobei die zwei Führungsstrukturen (80) eine Vielzahl von Führungslöchern (81), die in dem ersten, dem zweiten, dem dritten und dem vierten Expansionsblock (11, 12, 13, 14) quer ausgebildet sind, und zwei Führungselemente (82) umfassen, wobei die zwei Führungselemente (82) zwischen dem ersten und dem dritten Expansionsblock (11, 13) bzw. zwischen dem zweiten und dem vierten Expansionsblock (12, 14) angeordnet sind, wobei jedes der Führungselemente (82) mindestens eine Führungsstange (820) umfasst und zwei Enden der Führungsstange (820) teleskopartig in entsprechende zwei der Führungslöcher (81) eingesetzt sind.

12. Expandierbare Vorrichtung mit mehreren Abschnitten nach Anspruch 10, wobei die zwei Führungsstrukturen (80) eine Vielzahl von Führungslöchern (81), die in dem ersten, dem zweiten, dem dritten und dem vierten Expansionsblock (11, 12, 13, 14) quer ausgebildet sind, und zwei Führungselemente (82) umfassen, wobei die zwei Führungselemente (82) zwischen dem ersten und dem dritten Expansionsblock (11, 13) bzw. zwischen dem zweiten und dem vierten Expansionsblock (12, 14) angeordnet sind, wobei jedes der Führungselemente (82) zwei parallele Führungsstangen (820) und eine Verbindungsstange (821) umfasst, die zwischen zentralen Abschnitten der zwei Führungsstangen (820) verbunden ist, und jeweilige zwei Enden der zwei Führungsstangen (820) teleskopartig in die Vielzahl von Führungslöchern (81) eingesetzt sind.

## Revendications

1. Dispositif expansible à plusieurs sections, comprenant :
un module d'expansion (10) pouvant s'expanser longitudinalement et latéralement ;
un premier élément de poussée (20), disposé au niveau d'une extrémité avant du module d'expansion (10) et ayant un trou de vis (22) à l'intérieur de ce dernier ;
un second élément de poussée (30), disposé au niveau d'une extrémité arrière du module d'expansion (10) et ayant un trou débouchant (31) dans ce dernier ; et
un boulon (40) ayant une partie de vis (41) et une partie de tête (42), la partie de vis (41) étant insérée à travers le trou débouchant (31) du second élément de poussée (30) et vissé dans le trou de vis (22) du premier élément de poussée (20), la partie de tête (42) venant en butée contre un côté du second élément de poussée (30) opposé au premier élément de poussée (20),
dans lequel lorsque le boulon (40) est serré, le premier élément de poussée (20) et le second élément de poussée (30) sont poussés pour se rapprocher afin de pousser le module d'expansion (10) pour générer une expansion de premier étage et une expansion de second étage, l'expansion de premier étage permet au module d'expansion (10) de subir une expansion latéralement afin de régler sa largeur, l'expansion de second étage est réalisée après que le module d'expansion (10) a été latéralement expansé pour avoir une largeur maximum, de sorte que le module d'expansion (10) est expansé longitudinalement pour régler sa hauteur ;
dans lequel le module d'expansion (10) comprend un premier bloc d'expansion (11), un deuxième bloc d'expansion (12), un troisième bloc d'expansion (13), un quatrième bloc d'expansion (14), un premier bloc de guidage (15), un deuxième bloc de guidage (16), un troisième bloc de guidage (17) et un quatrième bloc de guidage (18) ; le deuxième bloc d'expansion (12) est positionné sous le premier bloc d'expansion (11), le troisième bloc d'expansion (13) est positionné à côté du premier bloc d'expansion (11), le quatrième bloc d'expansion (14) est positionné sous le troisième bloc d'expansion (13) ; le premier bloc de guidage (15) est disposé, de manière coulissante sur les faces d'extrémité avant des premier et deuxième blocs d'expansion (11, 12), le deuxième bloc de guidage (16) est disposé, de manière coulissante, sur les faces d'extrémité arrière des premier et deuxième blocs d'expansion (11, 12), les premier et deuxième blocs d'expansion (11, 12) sont déplacés vers le haut et vers le bas entre les premier et deuxième blocs de guidage (15, 16) pour s'expanser longitudinalement ; le troisième bloc de guidage (17) est disposé, de manière coulissante, sur les faces d'extrémité avant des troisième et quatrième blocs d'expansion (13, 14), le quatrième bloc de guidage (18) est disposé, de manière coulissante, sur les faces d'extrémité arrière des troisième et quatrième blocs d'expansion (13, 14), les troisième et quatrième blocs d'expansion (13, 14) sont déplacés vers le haut et vers le bas entre les troisième et quatrième blocs de guidage (17, 18) pour s'expanser longitudinalement ; le premier élément de poussée (20) est disposé de manière coulissante sur les faces d'extrémité avant des premier et troisième blocs de guidage (15, 17), le second élément de poussée (30) est disposé,
de manière coulissante, sur les faces d'extrémité arrière des deuxième et quatrième blocs de guidage (16, 18), les premier et deuxième blocs de guidage (15, 16) et les troisième et quatrième blocs de guidage (17, 18) entraînent respectivement les premier et deuxième blocs d'expansion (11, 12) et les troisième et quatrième blocs d'expansion (13, 14) pour se déplacer vers la gauche et vers la droite entre les premier et second éléments de poussée (20, 30) pour s'expanser latéralement ;
dans lequel le premier bloc d'expansion (11), le deuxième bloc d'expansion (12), le troisième bloc d'expansion (13) et le quatrième bloc d'expansion (14) comprennent chacun un bloc avant (a) et un bloc arrière (b), le bloc avant (a) et le bloc arrière (b) sont raccordés, de manière coulissante, entre eux, avec des faces de raccordement inclinées (a1, b1) correspondantes, les blocs avant (a) et les blocs arrière (b) des premier, deuxième, troisième et quatrième blocs d'expansion (11, 12, 13, 14) peuvent s'expanser longitudinalement pour se coupler avec les faces impaires opposées des vertèbres supérieures et inférieures (50, 51) afin de former l'expansion de second étage ;
dans lequel les faces de raccordement inclinées (a1, b1) correspondantes sont prévues avec une rainure à queue d'aronde (b2) et un rail de guidage (a2) destinés à être mis en prise entre eux.

2. Dispositif expansible à plusieurs sections selon la revendication 1, dans lequel :
des extrémités avant et arrière du premier bloc d'expansion (11) sont formées avec deux premiers plans inclinés (110) qui sont respectivement inclinés vers l'extérieur de bas en haut ;
des extrémités avant et arrière du deuxième bloc d'expansion (12) sont formées avec deux deuxièmes plans inclinés (120) qui sont respectivement inclinés vers l'extérieur de haut en bas ;
des extrémités avant et arrière du troisième bloc d'expansion (13) sont formées avec deux troisièmes plans inclinés (130) qui sont respectivement inclinés vers l'extérieur de bas en haut ;
des extrémités avant et arrière du quatrième bloc d'expansion (14) sont formées avec deux quatrièmes plans inclinés (140) qui sont respectivement inclinés vers l'extérieur de haut en bas ;
une extrémité arrière du premier bloc de guidage (15) est formée avec un premier plan incliné supérieur (150) et un premier plan incliné inférieur (151) correspondant aux premier et deuxième plans inclinés (110, 120) sur les extrémités avant des premier et deuxième blocs d'expansion (11,12), une extrémité avant du premier bloc de guidage (15) est formée avec un premier plan incliné avant (154) qui est incliné vers l'extérieur de l'arrière vers l'avant ;
une extrémité avant du deuxième bloc de guidage (16) est formée avec un deuxième plan incliné supérieur (160) et un deuxième plan incliné inférieur (161) correspondant aux premier et deuxième plans inclinés (110, 120) sur les extrémités arrière des premier et deuxième blocs d'expansion (11, 12), une extrémité arrière du deuxième bloc de guidage (16) est formée avec un deuxième plan incliné arrière (164) qui est incliné vers l'extérieur de l'avant vers l'arrière ;
une extrémité arrière du troisième bloc de guidage (17) est formée avec un troisième plan incliné supérieur (170) et un troisième plan incliné inférieur (171) correspondant aux troisième et quatrième plans inclinés (130, 140) sur les extrémités avant des troisième et quatrième blocs d'expansion (13, 14), une extrémité avant du troisième bloc de guidage (17) est formée avec un troisième plan incliné avant (174) qui est incliné vers l'extérieur de l'arrière vers l'avant ;
une extrémité avant du quatrième bloc de guidage (18) est formée avec un quatrième plan incliné supérieur (180) et un quatrième plan incliné inférieur (181) correspondant aux troisième et quatrième plans inclinés (130,140) sur les extrémités arrière des troisième et quatrième blocs d'expansion (13, 14), une extrémité arrière du quatrième bloc de guidage (18) est formée avec un quatrième plan incliné arrière (184) qui est incliné vers l'extérieur de l'avant vers l'arrière ;
deux côtés du premier élément de poussée (20) ont deux premiers plans inclinés d'élément de poussée (23) correspondant aux premier et troisième plans inclinés avant (154, 174) ;
deux côtés du second élément de poussée (30) ont deux deuxièmes plans inclinés d'élément de poussée (32) correspondant aux deuxième et quatrième plans inclinés arrière (164, 184) ;
des structures de mise en prise concave et convexe qui peuvent être mises en prise, de manière coulissante, entre elles, sont disposées entre les deux premiers plans inclinés (110) et les premier et deuxième plans inclinés supérieurs (150, 160), entre les deux deuxièmes plans inclinés (120) et les premier et deuxième plans inclinés inférieurs (151, 161), entre les deux troisièmes plans inclinés (130) et les troisième et quatrième plans inclinés supérieurs (170, 180), entre les deux quatrièmes plans inclinés (140) et les troisième et quatrième plans inclinés inférieurs (171, 181), entre les deux premiers plans inclinés d'élément de poussée (23) et les premier et troisième plans inclinés avant (154, 174) et entre les deux deuxièmes plans inclinés d'élément de poussée (32) et les deuxième et quatrième plans inclinés arrière (164, 184), respectivement.

3. Dispositif expansible à plusieurs sections selon la revendication 2, dans lequel deux premiers rails de guidage de plan incliné (111) sont respectivement disposés sur les deux premiers plans inclinés (110) ; deux deuxièmes rails de guidage de plan incliné (121) sont respectivement disposés sur les deux deuxièmes plans inclinés (120) ; deux deuxièmes rails de guidage de plan incliné (113) sont respectivement inclinés sur les deux deuxièmes plans inclinés (130) ; deux quatrièmes rails de guidage de plan incliné (141) sont respectivement disposés sur les deux quatrièmes plans inclinés (140) ; le premier plan incliné supérieur (150) est prévu avec une première rainure supérieure à queue d'aronde (152) pour le premier rail de guidage de plan incliné (111) sur l'extrémité avant du premier bloc d'expansion (111) à insérer dans cette dernière, le premier plan incliné inférieur (151) est prévu avec une première rainure inférieure à queue d'aronde (153) pour le deuxième rail de guidage de plan incliné (121) sur l'extrémité avant du deuxième bloc d'expansion (12) à insérer dans cette dernière, le premier plan incliné avant (154) est prévu avec une première rainure avant à queue d'aronde (155) ; le deuxième plan incliné supérieur (160) est prévu avec une deuxième rainure supérieure à queue d'aronde (162) pour le premier rail de guidage de plan incliné (111) sur l'extrémité arrière du premier bloc d'expansion (11) à insérer dans cette dernière, le deuxième plan inférieur incliné (161) est prévu avec une deuxième rainure inférieure à queue d'aronde (163) pour le deuxième rail de guidage de plan incliné (121) sur l'extrémité arrière du deuxième bloc d'expansion (12) à insérer dans cette dernière, le deuxième plan incliné arrière (164) est prévu avec une deuxième rainure arrière à queue d'aronde (165) ; le troisième plan incliné supérieur (170) est prévu avec une troisième rainure supérieure à queue d'aronde (172) pour le troisième rail de guidage de plan incliné (131) sur l'extrémité avant du troisième bloc d'expansion (13) à insérer dans cette dernière, le troisième plan incliné inférieur (171) est prévu avec une troisième rainure inférieure à queue d'aronde (173) pour le quatrième rail de guidage de plan incliné (141) sur l'extrémité avant du quatrième bloc d'expansion (14) à insérer dans cette dernière, le troisième plan incliné avant (174) est prévu avec une troisième rainure avant à queue d'aronde (175) ; le quatrième plan incliné supérieur (180) est prévu avec une quatrième rainure supérieure à queue d'aronde (182) pour le troisième rail de guidage de plan incliné (131) sur l'extrémité arrière du troisième bloc d'expansion (13) à insérer dans cette dernière, le quatrième plan incliné inférieur (181) est prévu avec une quatrième rainure inférieure à queue d'aronde (183) pour le quatrième rail de guidage de plan incliné (141) sur l'extrémité arrière du quatrième bloc d'expansion (14) à insérer dans cette dernière, le quatrième plan incliné avant (184) est prévu avec une quatrième rainure arrière à queue d'aronde (185) ; le deux premiers plans inclinés d'élément de poussée (23) sont prévus avec deux premiers rails de guidage d'élément de poussée (24) à insérer dans les première et troisième rainures avant à queue d'aronde (155, 175) ; et les deux deuxièmes plans inclinés d'élément de poussée (32) sont prévus avec deux deuxièmes rails de guidage d'élément de poussée (33) à insérer dans les deuxième et quatrième rainures arrière à queue d'aronde (165, 185).

4. Dispositif expansible à plusieurs sections selon la revendication 3, dans lequel deux premières pièces de butée avant (156) font saillie d'une extrémité inférieure du premier plan incliné avant (154), deux deuxièmes pièces de butée arrière (166) font saillie d'une extrémité inférieure du deuxième plan incliné arrière (164), deux troisièmes pièces de butée avant (176) font saillie d'une extrémité inférieure du troisième plan incliné avant (174), deux quatrièmes pièces de butée arrière (186) font saillie d'une extrémité inférieure du quatrième plan incliné arrière (184) ; le premier élément de poussée (20) a deux premières faces de butée (25) sur ses côtés supérieur et inférieur ; le second élément de poussée (30) a deux secondes faces de butée (34) sur ses côtés supérieur et inférieur ; lorsque les deux premières faces de butée (25) et les deux secondes faces de butée (34) sont respectivement bloquées par les deux premières pièces de butée avant (156), le deux troisièmes pièces de butée avant (176) et les deux deuxièmes pièces de butée arrière (166), les deux quatrièmes pièces de butée arrière (186), les premier et deuxième blocs d'expansion (11, 12) et les troisième et quatrième blocs d'expansion (13, 14) ne peuvent pas continuer à s'expanser latéralement, obtenant ainsi une largeur ultime de l'expansion de premier étage.

5. Dispositif expansible à plusieurs sections selon la revendication 1, dans lequel une tige (21) fait saillie d'un côté arrière du premier élément de poussée (20), et le trou de vis (22) est disposé dans la tige (21).

6. Dispositif expansible à plusieurs sections selon la revendication 5, dans lequel une extrémité de la tige (21) est couplée au premier élément de poussée (20) d'une manière vissée.

7. Dispositif expansible à plusieurs sections selon la revendication 1, dans lequel une surface inférieure du premier bloc d'expansion (11) a une pluralité de premières saillies de guidage (112) et une pluralité de premières rainures de guidage (113) qui sont agencées en quinconce et ont chacune une forme trapézoïdale ; une surface supérieure du deuxième bloc d'expansion (12) a une pluralité de deuxièmes rainures de guidage (122) et une pluralité de deuxièmes saillies de guidage (123) qui peuvent coulisser vers le haut et vers le bas par rapport à la pluralité de premières saillies de guidage (112) et à la pluralité de premières saillies de guidage (113) ; une surface inférieure du troisième bloc d'expansion (13) a une pluralité de troisièmes saillies de guidage (132) et une pluralité de troisièmes rainures de guidage (133) qui sont agencées en quinconce et ont chacune une forme trapézoïdale ; une surface supérieure du quatrième bloc d'expansion (14) a une pluralité de quatrièmes rainures de guidage (142) et une pluralité de quatrièmes saillies de guidage (143) qui peuvent coulisser vers le haut et vers le bas par rapport à la pluralité de troisièmes saillies de guidage (132) et à la pluralité de troisièmes rainures de guidage (133).

8. Dispositif expansible à plusieurs sections selon la revendication 3, dans lequel chacune parmi la première rainure avant à queue d'aronde (155), la deuxième rainure arrière à queue d'aronde (165), la troisième rainure avant à queue d'aronde (175) et la quatrième rainure arrière à queue d'aronde (185) a une ultime rainure de guidage (60) sur son fond, une extrémité de l'ultime rainure de guidage (60) est une extrémité ouverte, une autre extrémité de l'ultime rainure de guidage (60) est formée avec une face de butée ultime (61), une extrémité de chacun des deux premiers rails de guidage d'élément de poussée (24) et des deux deuxièmes rails de guidage d'élément de poussée (33) est prévue avec une partie relevée (62) à insérer dans l'ultime rainure de guidage (60), lorsque les quatre parties relevées (62) sont respectivement bloquées par les faces de butée ultimes (61) des quatre rainures de guidage ultimes (60), les premier et deuxième blocs d'expansion (11, 12) et les troisième et quatrième blocs d'expansion (13, 14) ne peuvent pas continuer à s'expanser latéralement, obtenant ainsi une largeur ultime de l'expansion de premier étage.

9. Dispositif expansible à plusieurs sections selon la revendication 4 ou 8, dans lequel chacune parmi la première rainure supérieure à queue d'aronde (152), la première rainure inférieure à queue d'aronde (153), la deuxième rainure supérieure à queue d'aronde (162), la deuxième rainure inférieure à queue d'aronde (163), la troisième rainure supérieure à queue d'aronde (172), la troisième rainure inférieure à queue d'aronde (173), la quatrième rainure supérieure à queue d'aronde (182) et la quatrième rainure inférieure à queue d'aronde (183) est prévue avec une rainure de guidage de limite (170) sur son fond, une extrémité de la rainure de guidage de limite (70) est une extrémité ouverte, une autre extrémité de la rainure de guidage de limite (70) est formée avec une face de mise en prise (71) ; une extrémité de chacun parmi les deux premiers rails de guidage de plan incliné (111), les deux deuxièmes rails de guidage de plan incliné (121), les deux troisièmes rails de guidage de plan incliné (131) et les deux quatrièmes rails de guidage de plan incliné (141) est prévue avec une partie de mise en prise (72) à insérer dans la rainure de guidage de limite (70) ; les huit parties de mise en prise (72) sont bloquées par les faces de mise en prise (71) des huit rainures de guidage de limite (70) pour empêcher les premier, deuxième, troisième et quatrième blocs d'expansion (11, 12, 13, 14) d'être dégagés des premier, deuxième, troisième et quatrième blocs de guidage (15, 16, 17, 18).

10. Dispositif expansible à plusieurs sections selon la revendication 1, comprenant en outre deux structures de guidage (80), les deux structures de guidage (80) étant respectivement disposées entre les premier et troisième blocs d'expansion (11, 13) et entre les deuxième et quatrième blocs d'expansion (12, 14) ; les deux structures de guidage (80) facilitent le déplacement synchrone des premier et troisième blocs d'expansion (11, 13) et des deuxième et quatrième blocs d'expansion (12, 14), lorsqu'ils sont expansés.

11. Dispositif expansible à plusieurs sections selon la revendication 10, dans lequel les deux structures de guidage (80) comprennent une pluralité de trous de guidage (81) qui sont transversalement formés dans les premier, deuxième, troisième et quatrième blocs d'expansion (11, 12, 13, 14) et deux éléments de guidage (82), les deux éléments de guidage (82) sont respectivement disposés entre les premier et troisième blocs d'expansion (11, 13) et entre les deuxième et quatrième blocs d'expansion (12, 14), chacun des éléments de guidage (82) comprend au moins une tige de guidage (820), et deux extrémités de la tige de guidage (820) sont insérées, par voie télescopique, dans deux trous correspondants des trous de guidage (81).

12. Dispositif expansible à plusieurs sections selon la revendication 10, dans lequel les deux structures de guidage (80) comprennent une pluralité de trous de guidage (81) qui sont formés, de manière transversale, dans les premier, deuxième, troisième et quatrième blocs d'expansion (11, 12, 13, 14) et deux éléments de guidage (82), les deux éléments de guidage (82) sont respectivement disposés entre les premier et troisième blocs d'expansion (11, 13) et entre les deuxième et quatrième blocs d'expansion (12, 14), chacun des éléments de guidage (82) comprend deux tiges de guidage (820) parallèles et une tige de raccordement (821) raccordées entre les parties centrales des deux tiges de guidage (820), et deux extrémités respectives des deux tiges de guidage (820) sont insérées, par voie télescopique, dans la pluralité de trous de guidage (81).
